# EUROPÄISCHE PATENTANMELDUNG

(11) **EP 2 808 394 A1**
(43) Veröffentlichungstag der Anmeldung: **03.12.2014**
(21) Anmeldenummer: 14166197.5
(22) Anmeldetag: 28.04.2014
(51) Int. Cl.: C12P 13/14, C12P 21/00, C12N 15/09

(54) **Mikroorganismus und Verfahren zur fermentativen Überproduktion von Gamma-Glutamylcystein und Derivaten dieses Dipeptids**

(30) Priorität: 17.05.2013 DE 102013209274
(71) Anmelder: Wacker Chemie AG, 81737 München (DE)
(72) Erfinder: Thön, Marcel, 81476 München (DE); Schlösser, Thomas, 84489 Burghausen (DE)
(74) Vertreter: Potten, Holger

(57) **Zusammenfassung**

Die Erfindung betrifft einen zur Überproduktion von γ-Glutamylcystein und dessen Derivaten bis-γ-Glutamylcystin und γ-Glutamylcystin befähigten prokaryotischen Mikroorganismenstamm, herstellbar aus einem Ausgangsstamm, dadurch gekennzeichnet, dass er im Vergleich zu dem Ausgangsstamm eine verminderte zelluläre Glutathion-Synthetase-Aktivität besitzt, und eine zelluläre γ-Glutamylcystein-Synthetase-Aktivität aufweist, die stärker erhöht ist als in einem Stamm mit gleichartig verminderter zellulärer Glutathion-Synthetase-Aktivität.

## Beschreibung

Die Erfindung betrifft einen prokaryotischen Mikroorganismenstamm, welcher zur Überproduktion von γ-Glutamylcystein (γGC) und den Derivaten dieses Dipeptids, γ-Glutamylcystin und bis-γ-Glutamylcystin geeignet ist, sowie Verfahren zur Herstellung dieser Verbindungen.

γ-Glutamylcystein ist ein Dipeptid, das in Zellen prokaryotischer und eukaryotischer Organismen durch Verknüpfung der Aminosäuren L-Cystein und L-Glutaminsäure entsteht.

Bis-γ-Glutamylcystin ist ein Disulfid, das bei der Oxidation von zwei Molekülen γ-Glutamylcystein entsteht. Diese Reaktion ist reversibel, was bedeutet, dass bis-γ-Glutamylcystin durch Reduktion (z.B. enzymatisch oder chemisch) wieder in γ-Glutamylcystein überführt werden kann.

γ-Glutamylcystin ist ein Disulfid, das bei der Oxidation von einem Molekül γ-Glutamylcystein mit einem Molekül L-Cystein entsteht. Diese Reaktion ist reversibel, was bedeutet, dass γ-Glutamylcystin durch Reduktion (z.B. chemisch) wieder in γ-Glutamylcystein und L-Cystein überführt werden kann.

Die Thiolverbindung γ-Glutamylcystein dient in zahlreichen Organismen als Vorstufe von Glutathion. Der erste Schritt der Glutathion-Biosynthese ist die Knüpfung einer γ-Peptidbindung zwischen der α-Aminogruppe von L-Cystein und der γ-Carboxygruppe von L-Glutaminsäure durch das ATP-abhängige Enzym γ-Glutamylcystein-Synthetase. Diese Reaktion stellt zudem den limitierenden Schritt der Glutathion-Biosynthese dar, da das Enzym γ-Glutamylcystein-Synthetase durch Glutathion gehemmt wird. Der zweite Schritt der Glutathion-Biosynthese ist die Reaktion von L-Glycin mit γ-Glutamylcystein zu Glutathion, wobei L-Glycin über eine α-Peptidbindung mit der Cysteinyl-Funktion von γ-Glutamylcystein verknüpft wird. Diese ebenfalls ATP-abhängige Reaktion wird durch das Enzym Glutathion-Synthetase katalysiert.

Aufgrund seiner antioxidativen Eigenschaften nimmt Glutathion eine Schlüsselrolle in den Zellen ein und ist als Reduktionsmittel, Co-Substrat und Co-Faktor an zahlreichen zellulären Prozessen beteiligt. Es sind aber auch Glutathion-defiziente Organismen bekannt, in denen die Glutathion-Vorstufe, γ-Glutamylcystein, die biologischen Aufgaben von Glutathion übernimmt. Als Beispiele sind Vertreter der halophilen Archaebakterien wie z.B. *Halobacterium halobium* genannt (Sundquist und Fahey, 1989, J. Biol. Chem. 264: 719-725).

In der Literatur sind zudem verschiedene Glutathion-defiziente Mikroorganismen-Stämme verschiedener Spezies beschrieben, z.B. von *Saccharomyces cerevisiae* (Grant et al., 1997, Mol. Biol. Cell. 8: 1699-1707), *Synechocystis* sp. (Cameron und Pakrasi, 2011, Plant Signal. Behav. 6: 89-92) oder *Eschericha coli* (Fuchs und Warner, 1975, J. Bacteriol. 124: 140-148). Diese Stämme besitzen Mutationen oder Deletionen im entsprechenden Glutathion-Synthetase-codierenden Gen (z.B. *gshB* für *E. coli* und *Synechocystis* sp. oder *gsh2* für *S. cerevisiae).* Durch Veränderung oder Verlust der Glutathion-Synthetase-Aktivität sind diese Stämme nicht mehr, oder nur in begrenztem Maße, in der Lage, Glutathion zu synthetisieren. Infolge dessen steigt der zelluläre γ-Glutamylcystein-Spiegel an. Diese mutierten Stämme sind lebensfähig, da das verstärkt gebildete und nun in erhöhter Konzentration vorliegende γ-Glutamylcystein zahlreiche Glutathion-spezifische Funktionen übernimmt. Sie zeichnen sich aber gegenüber den entsprechenden Wildtyp-Stämmen oftmals durch eine erhöhte Anfälligkeit gegenüber reaktiven Sauerstoffspezies und Schwermetallionen aus, sodass sie teilweise in ihrem Wachstum beeinträchtigt sind (Cameron und Pakrasi, 2011, Plant Signal. Behav. 6: 89-92; Helbig et al., 2008, J. Bacteriol. 190: 5439-5454).

Die Entgiftung von reaktiven Sauerstoffspezies, Schwermetallionen und Xenobiotika geschieht in vielen Organsismen, u.a. auch im Menschen, sehr häufig mit Hilfe von Glutathion (Grant et al., Mol. Biol. Cell. 8: 1699-1707). Ein niedriger Glutathion-Spiegel ist zudem oft ein Symptom und/oder Grund für verschiedene Krankheiten wie Autismus, Parkinson, Cystische Fibrose, HIV, Krebs oder Schizophrenie (Wu et al., 2004, J. Nutr. 134: 489-492). Daher besteht ein großes Interesse den intrazellulären Glutathion-Spiegel konstant zu halten oder gar zu erhöhen. γ-Glutamylcystein wurde bereits als vielversprechende Substanz zur Erhöhung des zellulären Glutathion-Spiegels erfolgreich getestet (Anderson und Meister, 1983, P.N.A.S. 80: 707-711).

WO2006102722 beschreibt ein enzymatisches Verfahren zur Herstellung von γ-Glutamylcystein, welches auf der Umsetzung von Cystein-Derivaten und einem γ-Glutamyldonor mit Hilfe einer immobilisierten γ-Glutamylcystein-Transpeptidase basiert.

Als mikrobielle γ-Glutamylcystein-Produzenten gelten hauptsächlich Pilzstämme der Ordnung Saccharomycetales, wie z.B. *Saccharomyces cerevisiae* oder *Candida utilis,* welche aber vorrangig für die Glutathion-Produktion genutzt werden. Derartige Stämme sind beispielsweise offenbart in WO2010116833A1, US7371557B2 und US2005074835A1. Andere Pilzstämme derselben Ordnung mit einer ausschließlich erhöhten γ-Glutamylcystein-Produktionsleistung sind u.a. in EP2251413A1, US7569360B2, US2004214308A1, US2003124684A1, US7410790B2 und EP1489173B1 beschrieben. Neben verschiedenen stammspezifischen Charakteristika, wie einer Cerulenin- bzw. nitroso-Guanidin-Resistenz oder einer Pantothensäure-Auxotrophie haben diese Pilzstämme als gemeinsames Merkmal eine reduzierte Glutathion-Synthetase-Aktivität.

Darüber hinaus beschreibt US2005042328A1 die intrazelluläre Anreicherung von γ-Glutamylcystein in einem C. *utilis*-Stamm mit reduzierter Glutathion-Synthetase-Aktivität und einer erhöhten gshl-Expression *(gshl* codiert für γ-Glutamylcystein-Synthetase).

Nachteile der pilzlichen γGC-Produktionssysteme sind die relativ niedrigen γ-Glutamylcystein-Ausbeuten, sowie die intrazelluläre Akkumulation von γ-Glutamylcystein, was einen Aufschluss der pilzlichen Zellen für eine mögliche Aufarbeitung von γ-Glutamylcystein notwendig machen würde.

Als prokaryotische Produzenten für γ-Glutamylcystein sind in US20100203592A1 verschiedene *E. coli*-Stämme beschrieben, welche alle eine erhöhte *gshA*-Expression aufweisen. Darüber hinaus besitzen diese Stämme eine normale oder erhöhte Glutathionsynthetase-Aktivität im Vergleich zum entsprechenden Ausgangsstamm. Im Gegensatz zu den pilzlichen Systemen ist mit diesen Stämmen eine Sekretion von γ-Glutamylcystein ins Kultivierungsmedium möglich. Die maximalen Ausbeuten mit diesen Stämmen liegen bei 262 mg/l, was für die Etablierung eines wirtschaftlichen Verfahrens nicht ausreichend ist.

Mikroorganismenstämme, welche zur Überproduktion der vielversprechenden bioaktiven Verbindung γ-Glutamylcystein und deren Derivaten bis-γ-Glutamylcystin und γ-Glutamylcystin befähigt sind, und die darüber hinaus die Sekretion dieser Verbindungen ins Kultivierungsmedium im g/l-Maßstab erlauben, existieren noch nicht.

Aufgabe der vorliegenden Erfindung ist es daher, einen Mikroorganismenstamm zur Verfügung zu stellen, der zur Überproduktion von γ-Glutamylcystein und dessen Derivaten bis-γ-Glutamylcystin und γ-Glutamylcystin befähigt ist und der darüber hinaus die extrazelluläre Akkumulation dieser Verbindungen im Kultivierungsmedium ermöglicht.

Die Aufgabe wird gelöst durch einen prokaryotischen Mikroorganismenstamm, der aus einem Ausgangsstamm herstellbar ist, und der im Vergleich zu dem Ausgangsstamm eine verminderte zelluläre Glutathion-Synthetase-Aktivität besitzt, und eine zelluläre γ-Glutamylcystein-Synthetase-Aktivität aufweist, die stärker erhöht ist, als in einem Stamm mit gleichartig verminderter zellulärer Glutathion-Synthetase-Aktivität.

Die Glutathion-Synthetase-Aktivität im erfindungsgemäßen Stamm ist vorzugsweise soweit vermindert, dass sie maximal 50% der Glutathion-Synthetase-Aktivität eines entsprechenden Wildtypstammes beträgt.

Bevorzugt sind prokaryotische Mikroorganismenstämme, die maximal 25% der Glutathion-Synthetase-Aktivität des Wildtypstammes vor Modifikation seiner Glutathion-Synthetase-Aktivität besitzen. Ganz besonders bevorzugt ist unter einer verminderten Glutathion-Synthetase-Aktivität eines Stammes das Fehlen der Glutathion-Synthetase-Aktivität in diesem Stamm zu verstehen.

Die γ-Glutamylcystein-Synthetase-Aktivität ist im erfindungsgemäßen Stamm vorzugsweise um mindestens den Faktor 5 höher als in einem Stamm mit gleichartig verminderter Glutathion-Synthetase-Aktivität.

Besonders bevorzugt sind im erfindungsgemäßen Mikroorganismenstamm sowohl die Glutathion-Synthetase-Aktivität als auch die γ-Glutamylcystein-Synthetase-Aktivität wie vorstehend beschrieben modifiziert.

Der Grad der DNA-Identitäten wird durch das Programm "nucleotide blast" zu finden auf der Seite http://blast.ncbi.nlm.nih.gov/ bestimmt, welches auf dem blastn-Algorithmus basiert. Als Algorithmus-Parameter für ein Alignment zweier oder mehrerer Nukleotidsequenzen wurden die voreingestellten Parameter genutzt. Die voreingestellten generellen Parameter sind: Max target sequences = 100; Short queries = "Automatically adjust parameters for short input sequences"; Expect Treshold = 10; Word size = 28; Automatically adjust parameters for short input sequences = 0. Die entsprechenden voreingestellten Scoring Parameter sind: Match/Mismatch Scores = 1,-2; Gap Costs = Linear.

Für den Vergleich von Proteinsequenzen wird das Programm "protein blast", auf der Seite http://blast.ncbi.nlm.nih.gov/, genutzt. Dieses Programm greift auf den blastp-Algorithmus zurück. Als Algorithmus-Parameter für ein Alignment zweier oder mehrerer Proteinsequenzen wurden die voreingestellten Parameter genutzt. Die voreingestellten generellen Parameter sind: Max target sequences = 100; Short queries = "Automatically adjust parameters for short input sequences"; Expect Treshold = 10; Word size = 3; Automatically adjust parameters for short input sequences = 0. Die voreingestellten Scoring Parameter sind: Matrix = BLOSUM62; Gap Costs = Existence: 11 Extension: 1; Compositional adjustments = Conditional compositional score matrix adjustment.

Als Ausgangsstämme für die Generierung der erfindungsgemäßen Mikroorganismenstämme sind prinzipiell alle prokaryotischen Mikroorganismenstämme geeignet, die den Biosyntheseweg für γ-Glutamylcystein aufweisen, und die durch Fermentation kultivierbar sind. Solche Mikroorganismen können den Domänen der Bacteria (ehemals Eubacteria) oder Archaea (ehemals Archaebacteria) angehören.

Bevorzugt handelt es sich bei diesen Organismen um Vertreter der phylogenetischen Gruppe der Bacteria. Besonders bevorzugt sind Mikroorganismen der Familie der Enterobacteriaceae, insbesondere die Spezies *Escherichia coli* und *Pantoea ananatis.*

Die γ-Glutamylcystein-Synthetase-Aktivität wird anhand der γ-Glutamylcystein-Bildung über die Zeit gemessen, wie in Beispiel 9 beschrieben.

Die Glutathion-Synthetase-Aktivität wird anhand der Bildungsrate von ADP mit einem Pyruvatkinase-gekoppelten Enzymtest bestimmt, wobei L-Glycin und L-γ-Glutamyl-L-α-Aminobutyrat als Substrate umgesetzt werden (Kim et al., 2003, J. Biochem. Mol. Biol. 36: 326-331).

Als Ausgangsstämme für die Generierung der erfindungsgemäßen Mikroorganismen eignen sich bevorzugt prokaryotische Mikroorganismenstämme, die den Biosyntheseweg für γ-Glutamylcystein aufweisen und darüber hinaus eine, im Vergleich zu einem entsprechenden Ausgangsstamm, erhöhte L-Cysteinbiosyntheseleistung besitzen. Eine erhöhte L-Cysteinbiosyntheseleistung ist für die Bildung von γ-Glutamylcystein von Vorteil, da die Bereitstellung ausreichender Mengen an L-Cystein als γ-GlutamylcysteinVorstufe für eine hohe γ-Glutamylcystein-Produktion nicht limitierend sein sollte. Unter einer "erhöhten L-Cystein-Biosyntheseleistung" versteht man im Rahmen der Erfindung die Fähigkeit eines Mikroorganismenstammes, mehr L-Cystein oder dessen Derivate L-Cystin und Thiazolidin zu produzieren als ein entsprechender Wildtyp-, Ausgangs-, oder nicht-modifizierter Stamm. Dies äußert sich typischerweise in einer Anreicherung von L-Cystein, L-Cystin und/oder Thiazolidin im Medium. Eine erhöhte L-Cystein-Biosyntheseleistung liegt demnach vor, wenn während oder am Ende einer, dem Fachmann bekannten, Fermentation des entsprechenden Mikroorganismenstammes mindestens 0,5 g/l "Gesamtcystein" (L-Cystein + L-Cystin + Thiazolidin) im Medium nachgewiesen werden können.

Potentielle Ausgangsstämme mit einer erhöhten L-Cystein-Biosyntheseleistung, für die Generierung der erfindungsgemäßen Mikroorganismen, sind beispielsweise offenbart in US20040038352A1, US20090053778, EP1528108A1, EP2345667A2 oder EP2138585.

Darüber hinaus sind bereits Gene oder Varianten dieser Gene (Allele) im Stand der Technik bekannt, deren Einsatz zur Überproduktion der Aminosäure L-Cystein und/oder den Vorstufen L-Serin bzw. O-Acetyl-Serin führen:
- serA-Allele wie beschrieben in EP0620853B1, EP1496111B1 und EP0931833A2:
   Diese *serA*-allele codieren für 3-Phosphoglycerat-Dehydrogenasen, die einer verminderten Feedback-Hemmung durch L-Serin unterliegen. Dadurch wird die Bildung von 3-Hydroxypyruvat weitgehend vom Serin-Spiegel der Zelle entkoppelt, was einen besseren Stofffluss zum L-Cystein erlaubt.
- cysE-Allele, wie beschrieben in WO9715673; Nakamori S. et al., 1998, Env. Microbiol 64: 1607-1611 oder Takagi H. et al., FEBS Lett. 452: 323-327:
   Diese *cysE*-Allele codieren für Serin-O-Acetyl-Transferasen, die einer verminderten Feedback-Hemmung durch L-Cystein unterliegen. Dadurch wird die Bildung von O-Acetyl-Serin und L-Cystein weitgehend vom L-Cystein-Spiegel der Zelle entkoppelt.
- Effluxgene wie beschrieben in EP885962A1:
   Der in EP885962A1 beschriebene orf306 wurde im Lauf der Zeit mehrfach annotiert. Die DNA-Sequenz des als orf306 oder auch orf299, *ydeD* und *eamA* bezeichneten Gens ist nun unter der Accession No. NC_004431 bzw. NC_007779 hinterlegt. Orf306 (orf299, *eamA* oder *ydeD)* codiert für ein Efflux-System, das zur Ausschleusung von Antibiotika und anderen toxischen Stoffen geeignet ist und die Überproduktion von L-Cystein, L-Cystin, N-Acetyl-Serin und/oder Thiazolidinderivaten bewirkt (Ohtsu I. et al., 2010, J. Biol. Chem. 285; 17479 - 17489; EP885962 und EP1233067B1).
- *cysB* beschrieben wie in DE19949579C1:
   Das *cysB*-Gen codiert für einen zentralen Regulator des Cysteinstoffwechsels und spielt u.a. eine entscheidende Rolle bei der Bereitstellung von Sulfid für die Cystein-Biosynthese.

Ein erfindungsgemäßer Mikroorganismenstamm exprimiert vorzugsweise auch eines oder mehrere der vorgenannten Gene bzw. Allele. Besonders bevorzugt wird der Einsatz eines der beschriebenen Allele von *cysE* oder *serA* sowie von orf306. Die *cysE-* und serA-Allele sowie orf306 können dabei einzeln oder zusammen im erfindungsgemäßen Mikroorganismenstamm exprimiert werden. Insbesondere bevorzugt weist ein erfindungsgemäßer Mikroorganismenstamm die in den Beispielen als erfindungsgemäß dargelegten, insbesondere die in Tab. 4 als erfindungsgemäß gekennzeichneten, genetischen Veränderungen auf.

Ein erfindungsgemäßer Mikroorganismenstamm kann mit Standardtechniken der Molekularbiologie erzeugt werden.

Die zelluläre Aktivität der γ-Glutamylcystein-Synthetase (GshA) kann zum Beispiel durch Erhöhung der Kopienzahl des *gshA-Gens* bzw. eines gshA-Homologs oder durch den Einsatz geeigneter Promotoren, welche zu einer verstärkten Expression des *gshA-Gens* bzw. eines gshA-Homolgs führen, erhöht werden.

Das *gshA-Gen* von *E. coli* codiert für das Enzym γ-Glutamylcystein-Synthetase (GshA). Das gshA-Gen ist charakterisiert durch SEQ ID No. 1. Das GshA-Genprodukt (GshA) ist charakterisiert durch SEQ ID No. 2. Im Rahmen der vorliegenden Erfindung sind gshA-Homologe Gene, die eine Sequenzidentität größer 30% zu SEQ ID No. 1 aufweisen. Besonders bevorzugt weisen *gshA-*Homologe eine Sequenzidentität größer 70% zu SEQ ID No. 1 auf.

GshA-Homologe sind Proteine mit einer Sequenzidentität größer 30% zu SEQ ID No. 2. Besonders bevorzugt weisen GshA-Homologe eine Sequenzidentität größer 70% zu SEQ ID No. 2 auf.

Die Erhöhung der Kopienzahl des gshA-Gens oder eines *gshA-*Homologs in einem Mikroorganismus kann mit dem Fachmann bekannten Methoden vorgenommen werden. So kann *gshA* oder ein *gshA-*Homolog zum Beispiel in Plasmid-Vektoren mit mehrfacher Kopienzahl pro Zelle (z.B. pBR322, pBR-Derivate, pBlueskript, pUC18, pUC19, pACYC184 und pACYC184-Derivate für *E. coli*) kloniert und in den Mikroorganismus eingebracht werden. Alternativ kann das gshA-Gen oder das gshA-Homolog mehrfach in das Chromosom eines Mikroorganismenstammes integriert werden. Als Integrationsverfahren können die bekannten Systeme mit temperenten Bakteriophagen, integrativen Plasmiden oder die Integration über homologe Rekombination genutzt werden (Hamilton et al., 1989, J. Bacteriol. 171: 4617-4622; Datsenko und Wanner, 2000, P.N.A.S. 97: 6640-6645).

Bevorzugt ist die Erhöhung der Kopienzahl durch Klonierung von *gshA* oder eines gshA-Homologs in Plasmid-Vektoren unter der Kontrolle eines Promotors. Besonders bevorzugt ist die Erhöhung der Kopienzahl in *E. coli* durch Klonierung von *gshA* oder eines *gshA*-Homologs in einem pACYC-Derivat wie z.B. pACYC184-LH (hinterlegt gemäß Budapester Vertrag bei der Deutschen Sammlung für Mikroorganismen und Zellkulturen, 83124 Braunschweig, Inhoffenstraße 7B am 18.08.1995 unter Nummer DSM 10172).

Unter einer Erhöhung der Kopienzahl ist vorzugsweise eine Erhöhung um mindestens den Faktor 10 zu verstehen.

Die Klonierung des *gshA*-Gens oder eines *gshA*-Homologs in Plasmid-Vektoren erfolgt beispielsweise durch spezifische Amplifikation mittels Polymerase-Ketten-Reaktion (PCR) unter Einsatz von spezifischen Primern, die das komplette Gen erfassen, und anschließender Ligation mit Plasmid-DNS-Fragmenten.

Als Kontrollregion für die Expression Plasmid-codierter Gene kann die natürliche Promotor- und/oder Operatorregion der Gene dienen.

Eine Erhöhung der Expressionsrate von *gshA* oder eines *gshA-*Homologs kann auch mittels anderer Promotoren erfolgen. Entsprechende Promotorsysteme wie beispielsweise in *E. coli* der konstitutive GAPDH-Promotor des gapA-Gens oder die induzierbaren lac-, tac-, trc, lambda-, ara- oder tet-Promotoren sind dem Fachmann bekannt (Markides S.C., 1996, Microbiol. Rev. 60: 512-538). Solche Konstrukte können in an sich bekannter Weise auf Plasmiden oder chromosomal verwendet werden.

Des Weiteren kann eine erhöhte zelluläre GshA-Aktivität dadurch erreicht werden, dass Translationsstartsignale, wie z.B. die Ribosomenbindestelle, oder die Shine-Dalgarno-Sequenz in optimierter Sequenz auf dem jeweiligen Konstrukt vorhanden sind, oder dass gemäß der "codon usage" seltene Codons gegen häufiger genutzte Codons ausgetauscht werden.

Die zelluläre GshA-Aktivität kann auch dadurch erhöht werden, dass eine Mutation (Substitution, Insertion oder Deletion einzelner oder mehrerer Nukleotide) in den Leserahmen des *gshA-*Gens oder eines *gshA*-Homologs eingebracht wird, welche dazu führt, dass die spezifische Aktivität von GshA oder eines GshA-Homologs erhöht wird. So führt beispielsweise der Austausch des ungewöhnlichen Startcodons TTG des gshA-Gens gegen das normale Startcodon ATG nicht nur zu einer erhöhten Expression von *gshA,* sondern auch zu einer Steigerung der zellulären Gesamtaktivität von GshA in *E. coli* (Kwak et al., 1998, J. Biochem. Mol. Biol. 31: 254-257).

Auch zielgerichtete Mutationen von *gshA,* welche auf Proteinebene einen Austausch von Alanin 494 gegen Valin oder Leucin (A494V oder A494L) bzw. eine Substitution von Serin an Position 495 gegen Threonin (S495T) zur Folge haben, führen in *E. coli* zu einer erhöhten zellulären GshA-Aktivität (Kwak et al., 1998, J. Biochem. Mol. Biol. 31: 254-257). Derartige Allele sind daher erfindungsgemäß bevorzugte gshA-Homologe.

Verfahren zur Verminderung der Glutathionsynthetase-Aktivität in einem Mikroorganismenstamm sind ebenfalls im Stand der Technik bekannt. Die zelluläre Glutathionsynthetase-Aktivität kann beispielsweise dadurch vermindert werden, dass eine Mutation (Substitution, Insertion oder Deletion einzelner oder mehrerer Nukleotide) in den Leserahmen des *gshB*-Gens oder eines *gshB-*Homologs eingebracht wird, welche dazu führt, dass die spezifische Aktivität von GshB oder eines GshB-Homologs vermindert wird. Dem Fachmann sind Verfahren zur Erzeugung solcher *gshB-*Allele bekannt. Allele des *gshB-*Gens kann man beispielsweise durch unspezifische oder gezielte Mutagenese mit der DNS des *gshB*-Wildtyp-Gens als Ausgangsmaterial herstellen. Beispiele solcher Allele, welche für GshB-Varianten mit einer verringerten GshB-Aktivität im Vergleich zum Wildtyp-Enzym codieren, sind beispielsweise in Kato et al. (1988, J. Biol. Chem. 263: 11646-11651) beschrieben.

Unspezifische Mutationen innerhalb des *gshB-Gens* oder des Promotorbereichs des *gshB-Gens* können z.B. durch chemische Agentien wie Nitrosoguanidin, Ethylmethansulfonsäure u.ä. und/oder durch physikalische Methoden und/oder durch unter bestimmten Bedingungen durchgeführte PCR-Reaktionen erzeugt werden. Methoden zur Einführung von Mutationen an spezifischen Positionen innerhalb eines DNS-Fragmentes sind bekannt. So kann beispielsweise der Austausch einer oder mehrerer Basen in einem DNS-Fragment, welches das *gshB-Gen* und dessen Promotorregion umfasst, mittels PCR unter Verwendung geeigneter Oligonukleotide als Primer erfolgen. Außerdem ist es möglich, das gesamte *gshB-*Gen bzw. ein neues *gshB-*Allel mittels Gensynthese herzustellen.

*gshB*-Allele werden in der Regel zunächst *in vitro* erzeugt und anschließend in das Chromosom der Zelle eingebracht, wodurch das ursprünglich vorhandene *gshB*-Wildtyp-Gen ersetzt und somit eine *gshB*-Mutante des Stammes erzeugt wird. *gshB*-Allele können mittels bekannter Standardmethoden in das Chromosom einer Wirtszelle anstelle des *gshB*-Wildtyp-Gens eingebracht werden. Dies kann beispielsweise mittels des in Link et al. (1997, J. Bacteriol. 179: 6228-37) beschriebenen Verfahrens zur Einführung chromosomaler Mutationen in ein Gen über den Mechanismus der homologen Rekombination erfolgen. Die chromosomale Deletion des gesamten *gshB*-Gens oder eines Teils davon ist beispielsweise mit Hilfe des λ-Red Rekombinase-Systems nach der von Datsenko und Wanner (2000, Proc. Natl. Acad. Sci. U S A. 97: 6640-5) beschriebenen Methode möglich. *gshB*-Allele können auch über eine Transduktion mittels P1-Phagen oder Konjugation aus einem Stamm mit *gshB*-Mutation auf einen *gshB*-Wildtyp-Stamm übertragen werden, wobei das *gshB*-Wildtyp-Gen im Chromosom gegen das entsprechende *gshB*-Allel ersetzt wird.

Darüber hinaus kann die Glutathionsynthetase-Aktivität einer Zelle auch dadurch verringert werden, dass mindestens ein für die Expressionsregulation notwendiges Element (z.B. Promotor, Enhancer, Ribosomen-Bindestelle) durch Substitution, Insertion oder Deletion einzelner oder mehrerer Nukleotide mutiert wird.

Das *gshB-Gen* von *E. coli* codiert für das Enzym Glutathion-Synthetase. Das *gshB-Gen* ist charakterisiert durch SEQ ID No. 3. Das GshB-Genprodukt (GshB) ist charakterisiert durch SEQ ID No. 4.

Im Rahmen der vorliegenden Erfindung sind *gshB*-Homologe Gene, die eine Sequenzidentität größer 30% zu SEQ ID No. 3 aufweisen. Besonders bevorzugt ist eine Sequenzidentität größer 70% zu SEQ ID No. 3. GshB-Homologe sind Proteine mit einer Sequenzidentität größer 30% zu SEQ ID No. 4. Besonders bevorzugt weisen GshB-Homologe eine Sequenzidentität größer 70% zu SEQ ID No. 4 auf.

Die Erfindung betrifft ferner ein Verfahren zur Überproduktion von γ-Glutamylcystein (γGC) und den Derivaten dieses Dipeptids, γ-Glutamylcystin und bis-γ-Glutamylcystin, welches dadurch gekennzeichnet ist, dass ein erfindungsgemäßer Mikroorganismus in einem Fermentationsmedium kultiviert wird, die Zellen aus dem Medium entfernt werden und die gewünschten Produkte aus dem Kulturmedium aufgereinigt werden.

Die Kultivierung (Fermentation) der für das erfindungsgemäße Verfahren notwendigen Mikroorganismen erfolgt im technischen Maßstab nach üblichen, dem Fachmann bekannten Fermentationsverfahren in einem Bioreaktor (Fermenter).

Die Fermentation findet vorzugsweise in einem üblichen Bioreaktor, beispielsweise einem Rührkessel, einem Blasensäulen-Fermenter oder einem Airlift-Fermenter statt. Besonders bevorzugt ist ein Rührkessel-Fermenter. Unter technischem Maßstab ist dabei eine Fermentergröße von mindestens 2 l zu verstehen. Bevorzugt sind Fermenter mit einem Volumen von mehr als 5 l, besonders bevorzugt Fermenter mit einem Volumen von > 50 l. Die Kultivierung der Zellen für eine γ-Glutamylcystein-Produktion wird unter aeroben Wachstumsbedingungen durchgeführt, wobei der Sauerstoff-Gehalt während der Fermentation bei maximal 50 % Sättigung eingestellt wird. Die Regulation der Sauerstoff-Sättigung in der Kultur erfolgt dabei automatisch über die Gaszufuhr und die Rührgeschwindigkeit.

Als Kohlenstoffquelle dienen vorzugsweise Zucker, Zuckeralkohole, organische Säuren oder zuckerhaltige Pflanzenhydrolysate. Besonders bevorzugt werden im erfindungsgemäßen Verfahren als Kohlenstoffquelle Glukose, Fruktose, Laktose, Glycerin oder Gemische, die zwei oder mehr dieser Verbindungen enthalten, eingesetzt.

Bevorzugt wird die Kohlenstoffquelle der Kultur so zudosiert, dass der Gehalt der Kohlenstoffquelle im Fermenter während der Produktionsphase 10 g/l nicht übersteigt. Bevorzugt ist eine maximale Konzentration von 2 g/l.

Als Stickstoff-Quelle werden im erfindungsgemäßen Verfahren vorzugsweise Ammoniak, Ammoniumsalze oder Proteinhydrolysate verwendet. Bei der Verwendung von Ammoniak als Korrekturmittel zur pH-Stabilisierung wird während der Fermentation regelmäßig diese Stickstoff-Quelle nachdosiert.

Als weitere Medienzusätze können Salze der Elemente Phosphor, Chlor, Natrium, Magnesium, Stickstoff, Kalium, Calcium, Eisen und in Spuren, d.h. in µM-Konzentrationen, Salze der Elemente Molybdän, Bor, Kobalt, Mangan, Zink und Nickel zugesetzt werden.

Des Weiteren können organische Säuren (z.B. Acetat, Citrat), Aminosäuren (z.B. L-Glutaminsäure, L-Cystein) und Vitamine (z.B. B1, B6) dem Medium zugesetzt werden.
L-Glutaminsäure kann dabei entweder direkt als Säure oder in Form eines ihrer Salze, wie z.B. Kalium- oder Natrium-Glutamat, einzeln oder als Gemisch, eingesetzt werden. Bevorzugt ist der Einsatz in Form von Kalium-Glutamat.

Als komplexe Nährstoffquellen können z.B. Hefeextrakt, Maisquellwasser, Sojamehl oder Malzextrakt zum Einsatz kommen.

Die Inkubationstemperatur für mesophile Mikroorganismen wie z.B. *E. coli* beträgt vorzugsweise 15 - 45 °C, besonders bevorzugt 30 - 37 °C.

Die Produktionsphase des erfindungsgemäßen Fermentationsverfahrens beginnt mit dem Zeitpunkt, ab dem erstmals γ-Glutamylcystein, bis-γ-Glutamylcystin oder γ-Glutamylcystin in der Kulturbrühe nachgewiesen werden kann. Typischerweise beginnt diese Phase ca. 8-12 h nach Inokulation des Produktionsfermenters mit einer Vorkultur.

Mikroorganismen, die nach dem beschriebenen Verfahren fermentiert werden, sezernieren in einem Batch- oder Fedbatch-Prozess nach einer Anwachsphase in einem Zeitraum von mindestens 48 h Stunden γ-Glutamylcystein und die davon abgeleiteten Verbindungen γ-Glutamylcystin und bis-γ-Glutamylcystin mit hoher Effizienz in das Fermentationsmedium.

Im Rahmen der Erfindung ist unter der Überproduktion von γ-Glutamylcystein vorzugsweise die Fähigkeit eines Mikroorganismusstammes zu verstehen, mehr γ-Glutamylcystein oder dessen Derivate γ-Glutamylcystin und bis-γ-Glutamylcystin zu produzieren als ein entsprechender Wildtyp-, Ausgangs-, oder nicht-modifizierter Stamm. Dies äußert sich typischerweise in einer Anreicherung von γ-Glutamylcystein, γ-Glutamylcystin und/oder bis-γ-Glutamylcystin im Medium. Eine Überproduktion von γ-Glutamylcystein liegt demnach vor, wenn am Ende einer Fermentation des entsprechenden Mikroorganismenstammes Ausbeuten von mindestens 0,2 g/l "Gesamt-γ-Glutamycystein" (γ-Glutamycystein + γ-Glutamycystin + bis-γ-Glutamylcystin) im Medium nachgewiesen werden können. Bevorzugt werden Ausbeuten an "Gesamt-γ-Glutamycystein" im Medium im Bereich von 3 bis 23 g/l, so wie sie beispielsweise mit den in den Tabellen 3 und 4 aufgeführten erfindungsgemäßen Mikroorganismen erzielt werden können. Besonders bevorzugt sind, hinsichtlich der Etablierung eines wirtschaftlichen Verfahrens, Ausbeuten im Bereich von 10 bis 23 g/l an "Gesamt-γ-Glutamycystein" im Medium.
Fig. 1 zeigt eine Restriktions- und Funktionskarte von Plasmid pACYC184-LH.
Fig. 2 zeigt eine Restriktions- und Funktionskarte von pgshAₚ-gshA^{TTG}.
Fig. 3 zeigt eine Restriktions- und Funktionskarte von ptufB.
Fig. 4 zeigt eine Restriktions- und Funktionskarte von ptufBₚ-gshA^{ATG}.
Fig. 5 zeigt eine Restriktions- und Funktionskarte von pgshA^{ATC}-serA2040.
Fig. 6 zeigt eine Restriktions- und Funktionskarte von pgshA^{ATC}-cysE14.
Fig. 7 zeigt eine Restriktions- und Funktionskarte von pgshA^{ATC}-cysE14-serA2040.
Fig. 8 zeigt eine Restriktions- und Funktionskarte von pgshA^{ATG}-serA2040-orf306.
Fig. 9 zeigt eine Restriktions- und Funktionskarte von pgshA^{ATG}-cysE14-orf306.
Fig. 10 zeigt eine Restriktions- und Funktionskarte von pgshA^{ATG}-cysE14-serA2040-orf306.
Fig. 11 zeigt HPLC-Chromatogramme von A) einer unbehandelten (oxidierten) Probe und B) einer mit DTT behandelten (reduzierten) Probe.

Die folgenden Beispiele dienen der weiteren Erläuterung der Erfindung.

### Beispiel 1: Deletion des Gens gshB (Inaktivierung Glutathion-Synthetase) im E. coli Stamm W3110 (ATCC27325)

Das Gen *gshB,* welches in *E. coli* für das Enzym Glutathion-Synthetase (GshB) codiert, wurde nach der von Datsenko und Wanner entwickelten "λ-Red-Methode" im *E. coli* Stamm W3110 (ATCC27325) deletiert (Datsenko und Wanner, 2000, P.N.A.S. 97: 6640-6645). Ein DNS-Fragment, welches für den Kanamycin-Resistenzmarker (kanR) codiert, wurde mit den Primern gshB-del-for (SEQ ID No. 5) und gshB-del-rev (SEQ ID No. 6) amplifiziert.

Der Primer gshB-del-for codiert für eine Sequenz, bestehend aus 30 Nukleotiden, welche homolog zum 5'-Ende des *gshB*-Gens ist, und eine 20 Nukleotide umfassende Sequenz, die komplementär zu einer DNS-Sequenz ist, welche eine der beiden FRT-sites (FLPrecognition target) auf dem Plasmid pKD13 (Coli Genetic Stock Center (CGSC) No. 7633) codiert. Der Primer gshB-del-rev codiert für eine Sequenz, bestehend aus 30 Nukleotiden, welche homolog zum 3'-Ende des *gshB-Gens* ist, und eine 20 Nukleotide umfassende Sequenz, die komplementär zu einer DNS-Sequenz ist, welche die zweite FRT-site auf dem Plasmid pKD13 codiert.

Das amplifizierte PCR-Produkt wurde, wie in Beispiel 2 von US5972663A beschrieben, mittels Elektroporation in den E-*scherichia coli* Stamm W3110 (ATCC27325) transformiert. Die Selektion der transformierten, *gshB*-defizienten Zellen erfolgte auf LB-Agarplatten, die 50 mg/l Kanamycin enthielten. Die Entfernung des Markergens (Kanamycin-Resistenz, kanR) erfolgte mit dem Enzym FLP-Rekombinase, welches auf dem Plasmid pCP20 (CGSC No. 7629) codiert ist. Aufgrund eines temperatursensitiven "origin of replication" (ori) kann das Plasmid pCP20 nach erfolgter Transformation durch Inkubation der *E. coli* Zellen bei 43°C wieder entfernt werden. Der auf diese Art und Weise generierte *E. coli gshB*-Deletionsstamm trägt die Bezeichnung W3110ΔgshB.

### Beispiel 2: Amplifikation des gshA-Gens mit eigenem Promotor

Die Promotorsequenz des *gshA-Gens* in *E. coli* ist bekannt (Watanabe et al., 1986, Nucleic Acids Res. 14: 4393-4400). Ein DNS-Fragment, welches für das *gshA*-Gen und den gshA-Promotor (gshAp) aus *E. coli* codiert, wurde mittels PCR amplifiziert. Das DNS-Fragment, welches für die Promotorsequenz des *gshA-Gens* codiert, wurde mit den Primern gshAp-gshA-for (SEQ ID No. 7) und gshAₚ-gshA-rev (SEQ ID No. 8) amplifiziert. Als Matrize für die PCR-Reaktion diente chromosomale DNS des *E. coli* Stammes W3110 (ATCC 27325).

Das etwa 1,9 kb große PCR-Fragment wurde über eine Agarose-Gelelektrophorese gereinigt und mit dem "QIAquick Gel Extraction Kit" (Qiagen GmbH, Hilden, D) nach Angaben des Herstellers aus dem Agarosegel isoliert. Anschließend wurde das gereinigte PCR-Fragment mit dem Restriktionsenzym XbaI verdaut und bei -20°C gelagert.

### Beispiel 3: Amplifizierung und Mutagenese des gshA-Gens

### A. Amplifizierung des gshA-Gens

Das Gen *gshA* aus *E. coli* wurde mittels PCR amplifiziert. Als Matrize für die PCR-Reaktion diente chromosomale DNS des E. *coli* Stammes W3110 (ATCC 27325). Für die Amplifikation wurde eine Taq-Polymerase (Qiagen GmbH) verwendet, die ein zusätzliches Adenin an das jeweilige 3'-Ende des PCR-Produkts anfügt. Im Zuge der Amplifikation von *gshA* wurde, durch den Einsatz eines geeigneten Primers, das ungewöhnliche Startcodon TTG gegen das Startcodon ATG ersetzt. Als spezifische Primer dienten die Oligonukleotide gshA-OE-for (SEQ ID NO. 9) und gshA-OE-rev (SEQ ID NO. 10)

Das resultierende 1,6 kb große DNS-Fragment wurde mit Hilfe einer Agarose-Gelelektrophorese gereinigt und mit dem "QIAquick Gel Extraction Kit" (Qiagen GmbH) aus dem Agarosegel isoliert. Die Ligation des amplifizierten und gereinigten PCR-Produkts mit dem Vektor pCR2.1-TOPO (Life Technologies, Life Technologies GmbH, Darmstadt, D) erfolgte über eine "TA-Klonierung" mit dem "TOPO^{®} TA Cloning^{®} Kit with PCR^{®}2.1 TOPO^{®}", nach Angaben des Herstellers (Life Technologies GmbH).

Der Ligationsansatz wurde in "DH5α™-T1R E. *coli-*Zellen" (Life Technologies) transformiert, in diesen Zellen vermehrt und nach einer Plasmidisolierung wurde die DNS-Sequenz der Plasmide mittels Sequenzierung verifiziert. Das resultierende Konstrukt mit gewünschter Sequenz trägt die Bezeichnung pCR2.1-gshA.

### B. Mutagenese des gshA-Gens

Für eine Umklonierung von *gshA* wurden zunächst die zwei störenden Restriktionsschnittstellen EcoRI und BglII im gshA-Gen mittels gerichteter Mutagenese entfernt. Die Mutagenesereaktionen wurden mit dem "GeneTailor™ Site-Directed Mutagenesis System" (Life Technologies GmbH) nach Angaben des Herstellers durchgeführt. Als Matrize für die erste Mutagenese diente das Plasmid pCR2.1-gshA. Für die Entfernung der EcoRI-Schnittstelle im gshA-Gen wurden die Mutageneseprimer gshA-EcoRImut-for (SEQ ID No. 11) und gshA-EcoRImut-rev (SEQ ID No. 12) verwendet. Im Anschluss an die durchgeführte Mutagenese der EcoRI-Schnittstelle im gshA-Gen wurde ein Teil der Mutagensereaktion erneut in "DH5α™-T1R *E*. *coli-*Zellen" der Firma Life Technologies transformiert und in diesen Zellen vermehrt. Nach einer Plasmidpräparation wurde die DNS-Sequenz der isolierten Plasmide mittels Sequenzierung verifiziert. Das resultierende Konstrukt mit richtiger DNS-Sequenz ohne EcoRI-Schnittstelle innerhalb des gshA-Gens trägt die Bezeichnung pCR2.1-gshA_mut1.

Für die Entfernung der BglII-Schnittstelle im *gshA*-Gen von pCR2.1-gshA_mut1 wurde ähnlich verfahren, nur dass die Mutageneseprimer gshA-BglIImut-for (SEQ ID No. 13) und gshA-BglIImut-rev (SEQ ID No. 14) für die Mutagenesereaktion verwendet wurden.

Ein Teil der zweiten Mutagenesereaktion wurde erneut in "DH5α™-T1R *E*. *coli-*Zellen" (Life Technologies GmbH) transformiert, in diesen Zellen vermehrt und nach einer erneuten Plasmidpräparation wurde die DNS-Sequenz der isolierten Plasmide mittels Sequenzierung verifiziert. Das resultierende Konstrukt mit richtiger DNS-Sequenz ohne EcoRI- und BglII-Schnittstellen innerhalb des *gshA-Gens* trägt die Bezeichnung pCR2.1-gshA_mut2.

### Beispiel 4: Amplifizierung des für den tufB-Promotor (ₚtufB) codierenden DNS-Fragments

Zur wirksamen Transkription (Überexpression) des gshA-Gens wurde die aktivierende Region des tRNA-tufB-Operons verwendet (Lee et al., 1981, Cell 25: 251-258). Dieses Operon codiert in E. *coli* für die Strukturgene *thrU, tyrU, glyT* und *thrT,* sowie für das Protein "Proteinketten-Elongations-Faktor EF-Tu" (TufB). Die gewünschte Promotorsequenz wurde mit den Oligonukleotiden tufBₚ-for (SEQ ID No. 15) und tufBₚ-rev (SEQ ID No. 16) amplifiziert. Als Matrize diente wiederum genomische DNS des Stammes *E. coli* W3110 (ATCC27325). Das erhaltene DNS-Fragment wurde über eine Agarose-Gelelektrophorese gereinigt und mit Hilfe des "QIAquick Gel Extraction Kit" (Qiagen GmbH) aus dem Agarosegel isoliert. Anschließend wurde das gereinigte PCR-Fragment mit dem Restriktionsenzym XbaI verdaut und bei -20°C gelagert.

### Beispiel 5: Konstruktion der Plasmide für eine gshA-Überexpression (gshA-Plasmide)

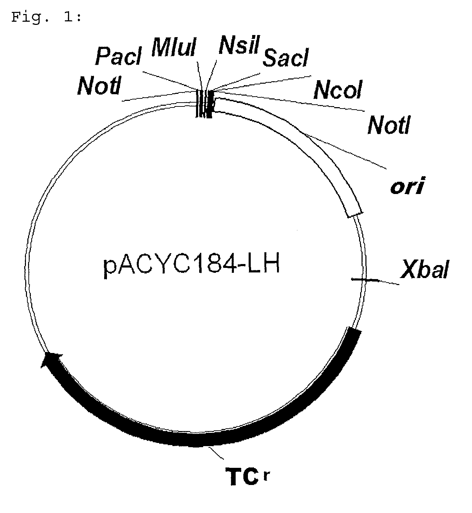

Als Basisplasmid für die Konstruktion der erfindungsgemäßen Plasmide wurde das Plasmid pACYC184-LH verwendet. Eine Restriktions- und Funktionskarte von Plasmid pACYC184-LH ist in Fig. 1 gezeigt. Das Plasmid pACYC184-LH codiert neben einer XbaI-Erkennungssequenz auch für eine Polylinkerregion mit folgenden Restriktionsschnittstellen:
NotI - NcoI - ScaI - NsiI - MluI - PacI - NotI

### A. Klonierung des gshA-Gens mit eigenem Promotor

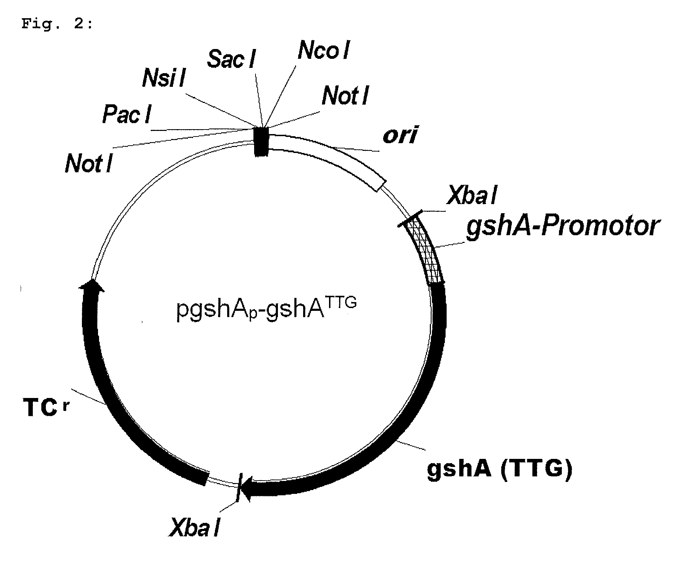

In die XbaI-Schnittstelle von pACYC184-LH wurde zunächst das in Beispiel 2 beschriebene, und mit XbaI verdaute PCR-Produkt kloniert, welches für das gshA-Gen mit eigenem Promotor und dem ungewöhnlichen Startcodon TTG codiert. Der Ligationsansatz wurde in "DH5α™-T1R E. coli-Zellen" (Life Technologies GmbH) transformiert, in diesen Zellen vermehrt und die DNS-Sequenz der isolierten Plasmide mittels Sequenzierung verifiziert. Das resultierende Konstrukt trägt die Bezeichnung pgshAₚ-gshA^{TTG} (siehe Fig. 2).

### B. Klonierung des tufB-Promotors

In die XbaI-Schnittstelle von pACYC184-LH wurde auch das in

Beispiel 4 beschriebene, und mit XbaI verdaute PCR-Produkt kloniert, welches für den tufB-Promotor (tufBₚ) codiert. Der Ligationsansatz wurde in "DH5α™-T1R E. *coli-Zellen"* (Life Technologies GmbH) transformiert, in diesen Zellen vermehrt und die DNS-Sequenz der isolierten Plasmide mittels Sequenzierung verifiziert. Das resultierende Konstrukt trägt die Bezeichnung pACYC184-tufBₚ.

### C. Optimierung des tufB-Promotors

Das Plasmid pACYC184-tufBp diente als Matrize für insgesamt 3 Optimierungen (Mutagenesen), die an der DNS-Sequenz des nativen tufB-Promotors vorgenommen wurden. Mit Hilfe des "GeneTailor™ Site-Directed Mutagenesis Systems" (Life Technologies GmbH) wurden die Ribosomenbindestelle (RBS), die -10 Region und die - 35 Region des tufB-Promotors dahingehend optimiert, dass sie letztlich für die entsprechenden Konsensussequenzen dieser prokaryotischen Promotorelemente codierten (siehe Tabelle 1).

**Tabelle 1: Vergleich der DNS-Sequenzen der RBS, -10 Region und der -35 Region des tufB-Promotors mit den entsprechenden Konsensussequenzen. Die mutagenisierten Nukleotide sind fett markiert.**

| **Element** | **im tufB-Promotor** | **Konsensussequenz** |
|---|---|---|
| RBS | 5'- A**CTT**GG - 3' | 5'- AGGAGG - 3' |
| -10 Region | 5'- TA**G**AAT - 3' | 5'- TATAAT - 3' |
| -35 Region | 5'- TTG**CAT** - 3' | 5'- TTGACA - 3' |

Für die Optimierung der RBS durch zielgerichtete Mutagenese des tufB-Promotors auf dem Plasmid pACYC184-tufBp wurden die Mutageneseprimer tufBₚ-RBSₒₚₜ-for (SEQ ID No. 17) und tufBₚ-RBSₒₚₜ-rev (SEQ ID No. 18) verwendet.

Im Anschluss an die durchgeführte Mutagenesereaktion der RBS des tufB-Promotors mit dem "GeneTailor™ Site-Directed Mutagenesis System" (Life Technologies) wurde ein Teil der Mutagensereaktion in "DH5α™-T1R *E*. *coli-*Zellen" (Life Technologies GmbH) transformiert, in diesen Zellen vermehrt und nach einer Plasmidpräparation wurde die DNS-Sequenz der isolierten Plasmide mittels Sequenzierung verifiziert. Das resultierende Konstrukt mit richtiger DNS- und optimierter RBS-Sequenz trägt die Bezeichnung pACYC184-tufBₚ-RBSₒₚₜ.

Für die Optimierung (Mutagenese) der -10 Region auf Basis von pACYC184-tufBₚ-RBSₒₚₜ wurde ähnlich verfahren, nur dass für die Mutagenese die Primer tufBₚ-10ₒₚₜ-for (SEQ ID No. 19) und tufBₚ-10ₒₚₜ-rev (SEQ ID No. 20) genutzt wurden.

Das entstandene und mittels Sequenzierung überprüfte Plasmid mit optimierter RBS und -10 Region trägt die Bezeichnung pACYC184-tufBp (RBS -10)ₒₚₜ.

Das Plasmid pACYC184-tufBₚ(RBS-10)ₒₚₜ diente wiederum als Matrize für die Mutagenese der -35 Region, welche mit den Primern tufBₚ-35ₒₚₜ-for (SEQ ID No. 21) und tufBₚ-35ₒₚₜ-rev (SEQ ID No. 22) durchgeführt wurde.

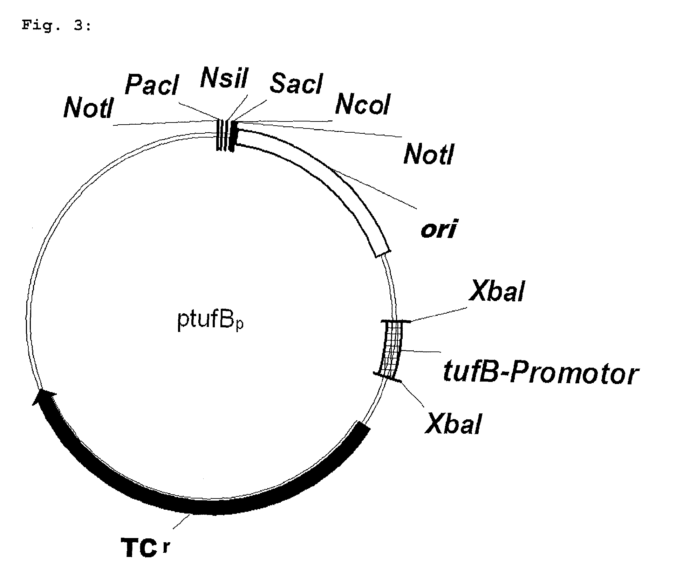

Das entstandene und mittels Sequenzierung überprüfte Plasmid mit optimierter RBS, -10 Region und -35 Region trägt die Bezeichnung ptufBₚ. Eine Restriktions- und Funktionskarte des resultierenden Plasmids ptufBₚ ist in Fig. 3 gezeigt.

### D. Klonierung des gshA-Gens mit optimiertem Startcodon hinter den optimierten tufB-Promotor in ptufBₚ

Das in Beispiel 3 beschriebene, mutagenisierte *gshA*-Gen mit dem Startcodon ATG wurde durch einen Restriktionsverdau mit den Enzymen EcoRI und BglII aus dem Plasmid pCR2.1-gshA_mut2 entfernt und in den mit EcoRI und BglII geschnittenen Vektor ptufBₚ umkloniert.

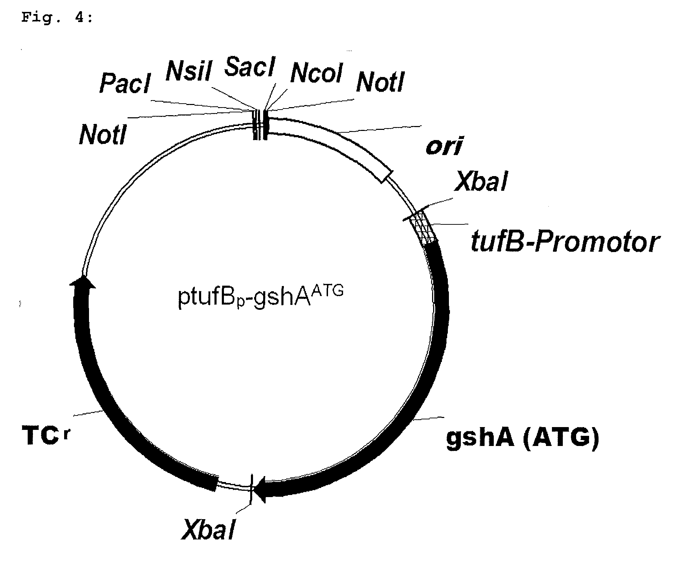

Das resultierende Konstrukt ptufBₚ-gshA^{ATG} (siehe Fig. 4) wurde in "DH5α™-T1R E. *coli*-Zellen" (Life Technologies GmbH) transformiert, in diesen Zellen vermehrt und die DNS-Sequenz der isolierten Plasmide mittels Sequenzierung verifiziert.

### Beispiel 6. Erweiterung des gshA-Expressions-Plasmids ptufBₚ-gshA^{ATG} um serA- und/oder cysE-Allele sowie orf306 (= orf299, ydeD bzw. eamA)

Um den Einfluss einer erhöhten L-Cystein-Biosynthese auf die γ-Glutamylcystein-Produktion zu untersuchen, wurde das Plasmid ptufBₚ-gshA^{ATG} um *serA-* und/oder *cysE*-Allele sowie den orf306 (= orf299, *eamA* oder *ydeD)* erweitert.

### A. Erweiterung von Plasmid ptufBₚ-gshA^{ATG} um serA-Allele

Für die Erweiterung des Plasmids ptufBₚ-gshA^{ATG} um ein bestimmtes *serA*-Allel wurde zunächst das *serA*-Gen mit eigenem Promotor über eine PCR amplifiziert. Als Matrize für die PCR-Reaktion diente chromosomale DNS des *E. coli* Stammes W3110 (ATCC 27325). Für die PCR-Reaktion wurden die Oligonukleotide serA-NcoI-for (SEQ ID. No. 23) und serA-SacI-rev (SEQ ID No. 24)verwendet. Das amplifizierte serA-Gen, inklusive eigenem Promotor, wurde anschließend mit den Enzymen NcoI und SacI verdaut und nach einer Reinigung über ein Agarosegel in den mit NcoI und SacI geschnittenen Vektor ptufBₚ-gshA^{ATG} ligiert.

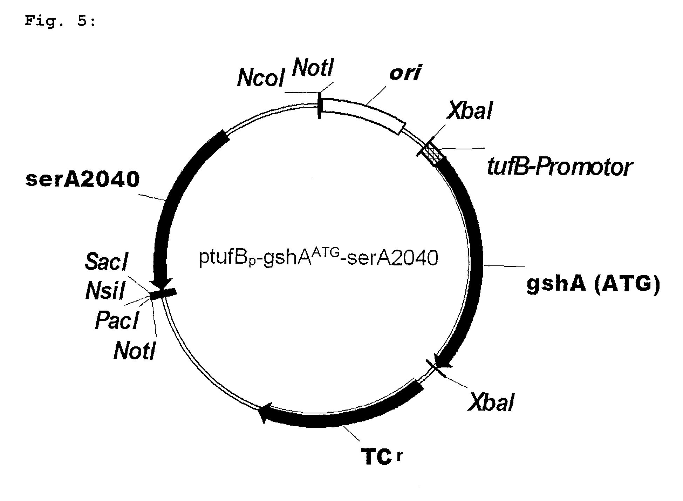

Der Ligationsansatz wurde in "DH5α™-T1R *E*. *coli-Zellen"* (Life Technologies GmbH) transformiert, in diesen Zellen vermehrt und die DNS-Sequenz der isolierten Plasmide mittels Sequenzierung verifiziert. Das aus dieser Klonierung hervorgegangene *gshA-*Plasmid mit serA unter Kontrolle des nativen Promotors wurde ptufBₚ-gshA^{ATG}-serA bezeichnet. Für die Generierung verschiedener serA-Allele, welche für gegenüber L-Serin feedbackresistente SerA-Varianten codieren, wurde wie in Beispiel 2 von EP1496111B1 verfahren, nur dass das Plasmid ptufBₚ-gshA^{ATG}-serA anstelle von pFL209 als Matrize für die Veränderung der Codons 349 und/oder 372 diente. Die aus den Mutagenesen hervorgegangenen *gshA*-Plasmide mit unterschiedlichen serA-Allelen wurden ptufBₚ-gshA^{ATG}-serA... bezeichnet, wobei ... der jeweiligen *serA-*Allelnummer entspricht (siehe z.B. Fig. 5 für ptufBₚ-gshA^{ATG}-serA2040).

### B. Erweiterung von Plasmid ptufBₚ-gshA^{ATG} um ein cysE-Allel

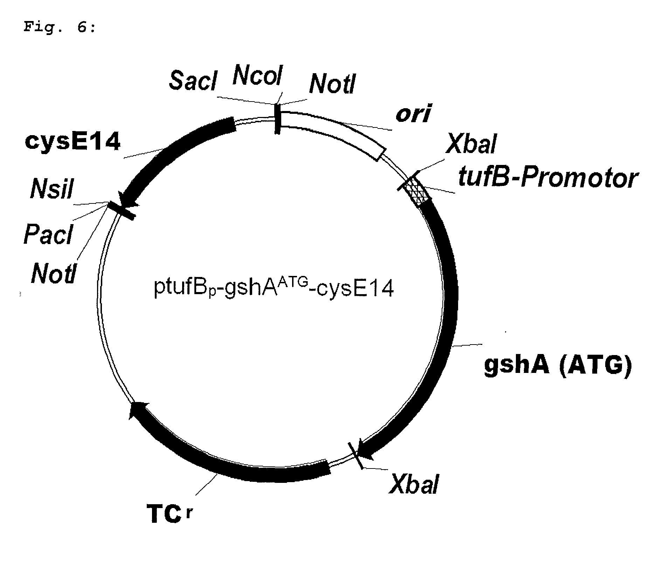

Für die Erweiterung des Plasmides ptufBₚ-gshA^{ATG} um ein bestimmtes *cysE*-Allel wurde zunächst eines der in Beispiel 6 (Tabelle No. 7) von WO9715673 offenbarten Plasmide pACYC184/cysE... mit NsiI und NcoI verdaut. Das jeweils etwa 1.0 kb große Fragment, welches für ein bestimmtes *cysE*-Allel unter Kontrolle des nativen *cysE*-Promotors codiert, wurde über ein Agarosegel gereinigt und anschließend in den mit NsiI und NcoI linearisierten Vektor ptufBₚ-gshA^{ATG} ligiert. Die aus dieser Klonierung hervorgegangenen *gshA*-Plasmide mit unterschiedlichen *cysE*-Allelen wurden ptufBₚ-gshA^{ATG}-cysE... bezeichnet, wobei ... der jeweiligen *cysE-*Allelnummer entspricht (siehe z.B. Fig. 6 für ptufBₚ-gshA^{ATG}-cysE14).

### C. Erweiterung der Plasmide ptufBₚ-gshA^{ATG}-serA... um ein cysE-Allel

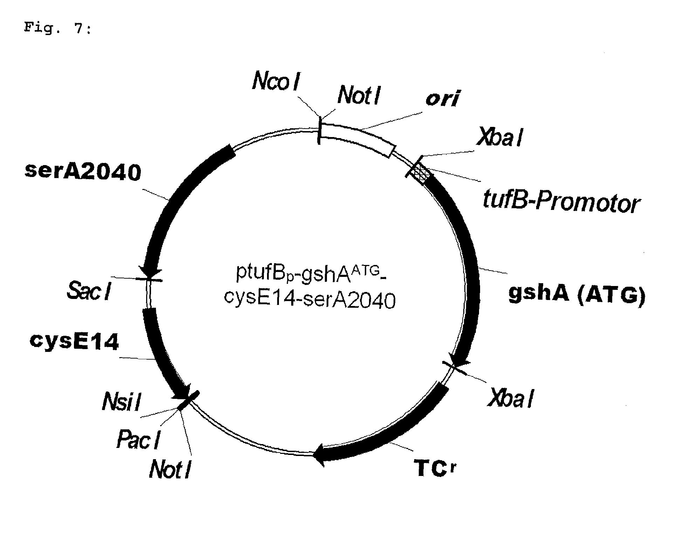

Für die Erweiterung der Plasmide ptufBₚ-gshA^{ATG}-serA... um ein bestimmtes *cysE*-Allel wurde zunächst eines der in Beispiel 6 (Tabelle No. 7) von WO9715673 offenbarten Plasmide pACYC184/cysE... mit SacI und NsiI verdaut. Das etwa 1.0 kb große Fragment wurde über ein Agarosegel gereinigt und anschließend in einen der mit SacI und NsiI linearisierten Vektoren ptufBₚ-gshA^{ATG}-serA... ligiert. Die aus dieser Klonierung hervorgegangenen *gshA*-Plasmide mit unterschiedlichen *cysE-* und *serA*-Allelen wurden ptufBₚ-gshA^{ATG}-cysE...-serA... bezeichnet, wobei ... der jeweiligen *cysE-*bzw. serA-Allelnummer entspricht (siehe z.B. Fig. 7 für ptufBₚ-gshA^{ATG}-cysE14-serA2040).

### D. Erweiterung der Plasmide ptufBₚ-gshA^{ATG}-serA..., ptufBₚ-gshA^{ATG}-cysE... bzw. ptufBₚ-gshA^{ATG}-cysE...-serA... um den orf306

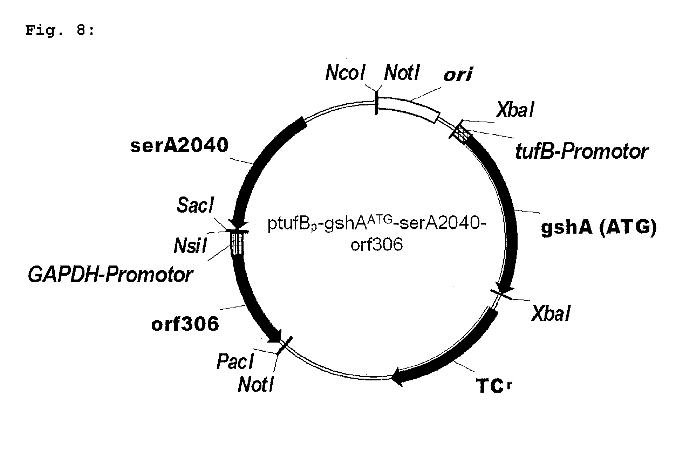

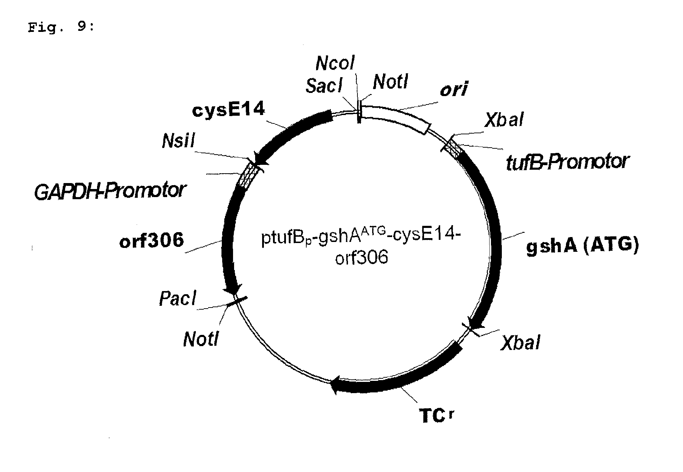

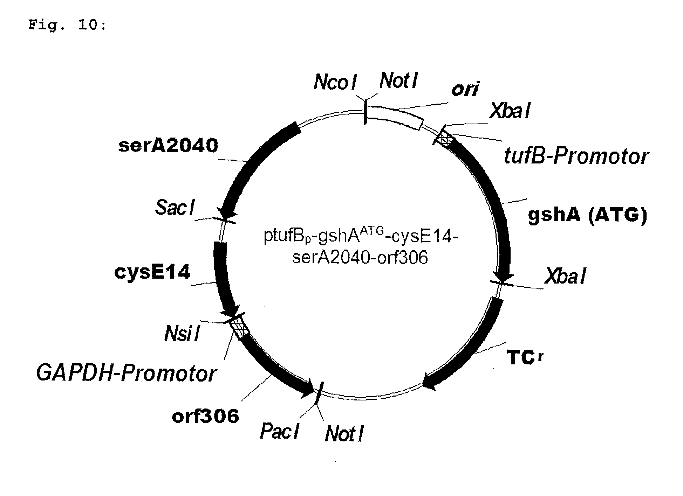

Für die Erweiterung der entsprechenden Plasmide ptufBₚ-gshA^{ATG}, ptufBₚ-gshA^{ATG}-serA..., ptufBₚ-gshA^{ATG}-cysE... bzw. ptufBₚ-gshA^{ATG}-cysE...-serA... um den orf306 wurde zunächst das in Beispiel 2 von EP885962B1 offenbarte Plasmid pACYC184/cysEIV-GAPDH-ORF306 mit NsiI und PacI verdaut. Das etwa 1.2 kb große Fragment, welches für den O-Acetylserin / Cysteine Exporter (EamA, YdeD) codiert, wurde über ein Agarosegel gereinigt und anschließend in einen der mit NsiI und PacI linearisierten Vektoren ptufBₚ- ptufBₚ-gshA^{ATG}-serA..., gshA^{ATG}-cysE... bzw. ptufBₚ-gshA^{ATG}-cysE...-serA... ligiert. Die aus dieser Klonierung hervorgegangenen *gshA*-Plasmide mit orf306 unter Kontrolles des GAPDH-Promotors wurden ptufBₚ-gshA^{ATG}-serA...-orf306, ptufBₚ-gshA^{ATG}-cysE...-orf306 bzw. ptufBₚ-gshA^{ATG}-cysE...serA...-orf306 bezeichnet, wobei ... der jeweiligen cy*sE-* bzw. serA-Allelnummer entspricht (siehe z.B. Fig. 8 für ptufBₚ-gshA^{ATG}-serA2040-orf306, Fig. 9 für ptufBₚ-gshA^{ATG}-cysE14-orf306 und Fig. 10 für ptufBₚ-gshA^{ATG}-cysE14-serA2040-orf306).

### Beispiel 7: Transformation der E. coli-Stämme W3110 (ATCC 27325) und W3110ΔgshB

Die *gshA*-Expressionsplasmide pgshAₚ-gshA^{TTG}, ptufBₚ-gshA^{ATG}, ptufBₚ-gshA^{ATG}-serA2040, ptufBₚ-gshA^{ATG}-cysE14, ptufBₚ-gshA^{ATG}-cysE14-serA2040, ptufBₚ-gshA^{ATG}-serA2040-orf306, ptufBₚ-gshA^{ATG}-cysE14-orf306 und ptufBₚ-gshA^{ATG}-cysE14-serA2040-orf306 sowie Plasmid pACYC184-LH als Negativkontrolle wurden, wie schon in Beispiel 1 beschrieben, mittels Elektroporation in die *E. coli* Stämme W3110 (ATCC27325) und W3110ΔgshB transformiert. Die Selektion auf Plasmid-tragende Zellen erfolgte auf LB-Agarplatten, die 15 mg/l Tetracyclin enthielten.

### Beispiel 8: Analytik von γ-Glutamylcystein und den Derivaten

Unter dem Begriff "Gesamt-γ-Glutamylcystein" sind γ-Glutamylcystein und die daraus gebildeten Oxidationsprodukte bis-γ-Glutamylcystin und γ-Glutamylcystin, die während der Fermentation entstehen und im Kulturüberstand akkumulieren, zusammengefasst. Die Konzentrationen an "Gesamt-γ-Glutamylcystein" wurden mittels HPLC-Analytik bestimmt.

### A. Vorbehandlung der Proben

Für die zu vermessenen Fermenterproben wurden zunächst die Mikroorganismen durch einen Zentrifugationsschritt und eine anschließenden Sterilfiltration von der Fermentationsbrühe getrennt.

Für die exakte Bestimmung der "Gesamt-γ-Glutamylcystein-Konzentration" wurden die γ-Glutamylcystein-Derivate in der zu vermessenden Probe 1 Stunde bei Raumtemperatur (22°C) mit einem molaren Überschuss an Dithiothreitol (DTT) reduziert. Da die reduzierende Wirkung von DTT nur bei neutralem bis alkalischem pH-Wert vollständig erfolgt, wurde der pH-Wert der Probe, wenn nötig, zuvor mit konzentrierter Kalilauge (KOH) auf einen pH-Wert > 7,0 eingestellt.

### B. HPLC-Analytik

Nach Ansetzen entsprechender Verdünnungen mit vollentsalztem Wasser erfolgte die HPLC-Analytik mit der Säule Synergi 4µm Hydro-RP 250 x 4,6 mm (Phenomenex Ltd., Aschaffenburg, D) bei 20°C. Als Fließmittel dienten 0,5% (v/v) Phosphorsäure (A) bzw. Acetonitril (B).

Zur Trennung von γ-Glutamylcystein aus einem zellfreien und reduzierten Substanzgemisch wurde folgende Methode für die HPLC genutzt.
- Flussrate = 0,5 ml/min
- Detektion bei 200 nm mit Diodenarray-Detektor (DAD)
- Die Trennung erfolgt isokratisch bei 100% A. Durch einen Spülschritt bei 100% B wird die Säule nach jedem Lauf gereinigt.

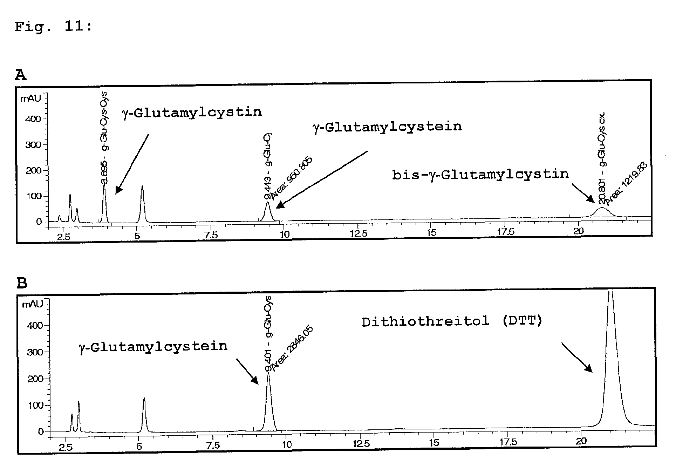

Als Referenzsubstanz, zur Bestimmung der Retentionszeit von γ-Glutamylcystein und der finalen Konzentration in den zu vermessenen Proben, diente reduziertes γ-Glutamylcystein der Firma Sigma Aldrich GmbH (Steinheim, D). Die Konzentration an "Gesamt-γ-Glutamylcystein" wurde anhand der Peakflächen im Chromatogramm berechnet (siehe Fig. 11).

### Beispiel 9: Bestimmung der γ-Glutamylcystein-Synthetase-Aktivität

Zur Bestimmung der γ-Glutamylcystein-Synthetase-Enzymaktivität wurden 30 ml SM1-Medium (12 g/l K₂HPO₄, 3 g/l KH₂PO₄, 5 g/l (NH₄)₂SO₄, 0,3 g/l MgSO₄ x 7 H₂O, 0,015 g/l CaCl₂ x 2 H₂O, 0,002 g/l FeSO₄ x 7 H₂O, 1 g/l Na₃Citrat x 2 H₂O, 0,1 g/l NaCl; 1 ml/l Spurenelementlösung, bestehend aus 0,15 g/l Na₂MoO₄ x 2H₂O, 2,5 g/l H₃BO₃, 0,7 g/l CoCl₂ x 6 H₂O, 0,25 g/l CuSO₄ x 5 H₂O, 1,6 g/l MnCl₂ x 4 H₂O, 0,3 g/l ZnSO₄ x 7 H₂O), das mit 15 g/l Glukose, 5 mg/l Vitamin B₁ und 15 mg/l Tetracyclin supplementiert wurde, mit einer 2 ml Übernachtkultur der in Tabelle 2 aufgeführten Stämme beimpft. Ausgehend von einer anfänglichen optischen Dichte bei 600 nm (OD₆₀₀) von 0,025 wurde der gesamte 30 ml-Ansatz für weitere 16 Stunden bei 30°C und 135 rpm inkubiert. Die Zellen wurden anschließend durch Zentrifugation geerntet, gewaschen und in 2 ml Puffer (100 mM Tris-HCl pH 8,2) resuspendiert. Der Zellaufschluss erfolgte mittels einer French Press (Spectronic Instruments, Inc. Rochester, NY, USA) bei einem Druck von 124106 kPa. Die Rohextrakte wurden durch Zentrifugation bei 30.000 g geklärt und die γ-Glutamylcystein-Synthetase-Aktivität anhand der Bildung von γ-Glutamylcystein über die Zeit gemessen. Das Reaktionsgemisch des zugrunde liegenden γ-Glutamylcystein-Bildungsassays setzt sich folgendermaßen zusammen:
100 mM Tris-HCl pH 8,2; 10 mM Kaliumglutamat; 10 mM L-Cystein; 20 mM MgCl₂; 150 mM KCl; 20 mM ATP und 0,25 bis 4 mg/ml Rohextrakt-Protein. Die ATP-abhängige Bildung von γ-Glutamylcystein aus L-Cystein und Kaliumglutamat wurde durch Zugabe von Rohextrakt-Protein gestartet. Über einen Zeitraum von mindestens 2 Stunden wurden Aliquots (20 µl bis 50 µl) dem Reaktionsgemisch entnommen. Anschließend wurde die γ-Glutamylcystein-Synthetase-Aktivität in den Proben (Aliquots) für 5 min bei 70°C inaktiviert und der Gehalt an gebildetem γ-Glutamylcystein mittels HPLC bestimmt (siehe Beispiel 8). Eine Unit an γ-Glutamylcystein-Synthetase-Aktivität entspricht dabei derjenigen Enzymmenge, die bei 25°C ein µmol γ-Glutamylcystein pro Minute bildet.

**Tabelle 2: γ-Glutamylcystein-Synthetase-Aktivitäten der untersuchten gshB-Deletionsstämme mit Plasmid-codierter gshA-Überexpression.**

| *E. coli*-Stamm | mU/mg Protein Rohextrakt |
|---|---|
| W3110ΔgshB/pACYC184-LH | 1,8 + 0,1 |
| W3110ΔgshB/pgshAₚ-gshA^{TTG} (*) | 9,1 ± 1,9 |
| W3110ΔgshB/ptufBₚ-gshA^{ATG} (*) | 143,2 ± 22,9 |

| | |
|---|---|
| ((*) erfindungsgemäßer Stamm) | |

### Beispiel 10: γ-Glutamylcystein-Produktion (Fermentation)

### A. Vorkultur 1 (Schüttelkolben):

100 ml LB-Medium mit 15 mg/l Tetracyclin wurden in einem 1 1-Erlenmeyerkolben mit Schikanen mit den in Tabellen 3 und 4 aufgelisteten *E*. *coli*-Stämmen von einer Agarkultur beimpft und für sieben Stunden auf einem Schüttler bei 130 rpm und 32°C inkubiert.

### B. Vorkultur 2 (Vorfermenter):

Ein Teil der jeweiligen Vorkultur 1 wurde in einen mit Fermentationsmedium gefüllten Fermenter des Typs Sixfors (Infors AG, Bottmingen, CH) überimpft, sodass zu Beginn eine optische Dichte von ca. 0,01 im Fermenter vorlag (gemessen bei 600 nm). Der verwendete Fermenter hatte ein Gesamtvolumen von 1 l und ein initiales Arbeitsvolumen von 0,7 l.

Das Fermentationsmedium enthielt folgende Bestandteile: 3 g/l (NH₄)₂SO₄, 1,7 g/l KH₂PO₄, 0,25 g/l NaCl, 0,6 g/l MgSO₄ x 7 H₂O, 0,03 g/l CaCl₂ x 2 H₂O, 0,15 g/l FeSO₄ x 7 H₂O, 1 g/l Na₃Citrat x 2 H₂O, 5 g/l Cornsteep Dry (CSD) und 3 ml/l Spurenelementlösung (bestehend aus 2,5 g/l H₃BO₃, 0,7 g/l CoCl₂ x 6 H₂O, 0,25 g/l CuSO₄ x 5 H₂O, 1,6 g/l MnCl₂ x 4 H₂O, 0,3 g/l ZnSO₄ x 7 H₂O, 0,15 g/l Na₂MoO₄ x 2 H₂O). Nach Sterilisation dieses Grundmediums wurden unter sterilen Bedingungen folgende Bestandteile zudosiert: 40 g/l Glukose, 0,018 g/l Vitamin B1, 0,09 g/l Vitamin B6 und 15 mg/l Tetracyclin.

Der pH-Wert im Fermenter wurde durch Zugabe einer 25%-igen NH₄OH-Lösung auf 7,0 eingestellt. Während der Fermentation wurde der pH-Wert durch automatische Korrektur mit 25% NH₄OH auf einem Wert von 7,0 gehalten. Die Kulturen wurden zu Beginn mit 400 rpm gerührt und mit einer Begasungsrate von 0,7 Volumen pro Volumen pro Minute (vvm) einer über einen Sterilfilter entkeimten Druckluft begast. Unter diesen Startbedingungen war die Sauerstoff-Sonde vor der Inokulation auf 100% Sättigung kalibriert worden. Der Soll-Wert für die O₂-Sättigung während der Fermentation wurde auf 50% eingestellt. Nach Absinken der O₂-Sättigung unter den Soll-Wert wurde eine Regulationskaskade gestartet, um die O₂-Sättigung wieder an den Soll-Wert heranzuführen. Dabei wurde zunächst die Gaszufuhr kontinuierlich erhöht (auf max. 1,4 vvm) und anschließend die Rührgeschwindigkeit auf maximal 1.200 rpm kontinuierlich gesteigert.

Die Fermentation wurde bei einer Temperatur von 32°C so lange durchgeführt, bis eine optische Dichte von 30 bis 40 erreicht wurde (gemessen bei 600 nm). Dies war in der Regel nach ca. 17 h der Fall.

### C. Hauptkultur (Hauptfermenter):

Die Fermentationen wurden in Fermentern des Typs BIOSTAT B-DCU der Firma Sartorius Stedim GmbH (Göttingen, D) durchgeführt. Es wurde ein Kulturgefäß mit 2 l Gesamtvolumen bei einem initialen Arbeitsvolumen von 1 l verwendet.

Das Fermentationsmedium enthält folgende Bestandteile: 3 g/l (NH₄)₂SO₄, 1,7 g/l KH₂PO₄, 0,25 g/l NaCl, 0,6 g/l MgSO₄ x 7 H₂O, 0,03 g/l CaCl₂ x 2 H₂O, 0,15 g/l FeSO₄ x 7 H₂O, 1 g/l Na₃Citrat x 2 H₂O, 15 g/l Cornsteep Dry (CSD) und 3 ml/l Spurenelementlösung (bestehend aus 2,5 g/l H₃BO₃, 0,7 g/l CoCl₂ x 6 H₂O, 0,25 g/l CuSO₄ x 5 H₂O, 1,6 g/l MnCl₂ x 4 H₂O, 0,3 g/l ZnSO₄ x 7 H₂O, 0,15 g/l Na₂MoO₄ x 2 H₂O). Nach Sterilisation dieses Grundmediums wurden unter sterilen Bedingungen folgende Bestandteile zudosiert: 10 g/l Glukose, 0,018 g/l Vitamin B1, 0,09 g/l Vitamin B6 und 15 mg/l Tetracyclin.

Zum Animpfen der Hauptkultur wurden 100 ml der Vorkultur 2 in das Fermentergefäß überführt. Der pH-Wert im Fermenter wurde zu Beginn durch Zugabe einer 25%-igen NH₄OH-Lösung auf 7,0 eingestellt und durch automatische Korrektur mit 25% NH₄OH auf diesem Wert gehalten. Die Kulturen wurden zu Beginn mit 400 rpm gerührt und mit einer Begasungsrate von 2 vvm einer über einen Sterilfilter entkeimten Druckluft begast. Unter diesen Startbedingungen war die Sauerstoff-Sonde vor der Inokulation auf 100% Sättigung kalibriert worden. Der Soll-Wert für die O₂-Sättigung während der Fermentation wurde auf 50% eingestellt. Nach Absinken der O₂-Sättigung unter den Soll-Wert wurde eine Regulationskaskade gestartet, um die O₂-Sättigung wieder an den Soll-Wert heranzuführen. Dabei wurde zunächst die Gaszufuhr kontinuierlich erhöht (auf max. 5 vvm) und anschließend die Rührgeschwindigkeit auf maximal 1.500 rpm kontinuierlich gesteigert. Die Fermentationen wurden bei einer Temperatur von 32°C durchgeführt. Sobald der Glukose-Gehalt im Fermenter von anfänglich 10 g/l auf ca. 2 g/l abgesunken war, erfolgte eine kontinuierliche Zudosierung einer 60% Glukose-Lösung. Die Fütterungsrate wurde so eingestellt, dass die Glukosekonzentration im Fermenter 2 g/l fortan nicht mehr überstieg. Die Glukose-Bestimmung wurde mit einem Glukoseanalysator der Firma YSI (Yellow Springs, Ohio, USA) durchgeführt.

Nach 5 h Fermentationszeit erfolgte die Zufütterung einer Schwefelquelle in Form einer sterilen 1,5 M (NH₄)₂SO₄-Stammlösung (Ammoniumsulfat) mit einer Rate von 8-17 mmol/l pro Stunde (Durchschnitt = 11 mmol/l pro Stunde).

Nach 16 h Fermentationszeit wurde der Hauptkultur zusätzlich Glutamat in Form einer sterilen 2,3 M Kaliumglutamat-Stammlösung mit einer Rate von 7,4 mmol/l pro Stunde zugesetzt. Die Fermentationsdauer betrug 72 Stunden. Nach 24 h, 48 h und 72 h wurden Proben entnommen und nach Reduktion mit DTT der Anteil an "Gesamt-γ-Glutamylcystein" im Kulturüberstand mittels HPLC-Analytik bestimmt.

**Tabelle 3: Einfluss der gshB-Deletion und/oder gshA-Überexpression auf die γ-Glutamylcystein-Produktion. Gehalt von "Gesamt-γ-Glutamylcystein" (γGC) nach Neutralisation der Fermenterbrühe und anschließender Reduktion der Oxidationsprodukte mit Dithiothreitol.**

| *E. coli*-Stamm | Gesamt-γ-Glutamylcystein (g/L) | | |
|---|---|---|---|
| | 24 h | 48 h | 72 h |
| W3110/pACYC184-LH | 0 | 0 | 0 |
| W3110/pgshAₚ-gshA^{TTG} | 0,2 ± 0,0 | 0,3 ± 0,0 | 0,3 ± 0,0 |
| W3110/ptufBₚ-gshA^{ATG} | 1,2 ± 0,0 | 1,4 ± 0,0 | 1,5 ± 0,1 |
| W3110ΔgshB/pACYC184-LH | 2,2 ± 0,1 | 2,7 ± 0,1 | 2,9 ± 0,2 |
| W3110ΔgshB/pgshAₚ-gshA^{TTG}(*) | 3,4 ± 0,2 | 5,4 ± 0,1 | 5,7 ± 0,3 |
| W3110ΔgshB/ptufBₚ-gshA^{ATG}(*) | 7,2 ± 0,3 | 13,1 ± 0,5 | 14,4 ± 0,3 |

| | | | |
|---|---|---|---|
| ((*) erfindungsgemäßer Stamm) | | | |

**Tabelle 4: Einfluss der gshB-Deletion und gshA-überexpression in Kombination mit einer erhöhten L-Cystein-Biosyntheseleistung auf die γ-Glutamylcystein-Produktion. Gehalt von "Gesamt-γ-Glutamylcystein" (γGC) nach Neutralisation der Fermenterbrühe und anschließender Reduktion der Oxidationsprodukte bis-γ-Glutamylcystin und γ-Glutamylcystin mit Dithiothreitol:**

| *E. coli* W3110ΔgshB mit Plasmid | Gesamt-γ-Glutamylcystein (g/L) | | |
|---|---|---|---|
| | 24 h | 48 h | 72 h |
| pgshAₚ-gshA^{TTG}(*) | 3,4 ± 0,2 | 5,4 ± 0,1 | 5,7 ± 0,3 |
| ptufBₚ-gshA^{ATG}(*) | 7,2 ± 0,3 | 13,1 ± 0,5 | 14,4 ± 0,3 |
| ptufBₚ-gshA^{ATG}-serA2040(*) | 8,5 ± 0,5 | 15,1 ± 1,1 | 16,5 ± 1,1 |
| ptufBₚ-gshA^{ATG}-cysE14(*) | 8,6 ± 0,3 | 15,2 ± 1,0 | 17,0 ± 0,9 |
| ptufBₚ-gshA^{ATG}-cysE14-serA2040(*) | 10,0 ± 0,7 | 16,6 ± 0,9 | 20,1 ± 1,2 |
| ptufBₚ-gshA^{ATG}-serA2040-orf306(*) | 8,7 ± 0,4 | 15,5 ± 0,2 | 16,9 ± 0,6 |
| ptufBₚ-gshA^{ATG}-cysE14-orf306(*) | 8,9 ± 0,5 | 15,7 ± 0,8 | 17,5 ± 1,1 |
| ptufBₚ-gshA^{ATG}-cysE14-serA2040-orf306(*) | 11,6 ± 0,3 | 17,9 ± 0,7 | 21,8 ± 1,0 |

| | | | |
|---|---|---|---|
| ((*) erfindungsgemäßer Stamm) | | | |

## Patentansprüche

1. Zur Überproduktion von γ-Glutamylcystein, bis-γ-Glutamylcystin und γ-Glutamylcystin befähigter prokaryotischer Mikroorganismenstamm, herstellbar aus einem Ausgangsstamm, **dadurch gekennzeichnet, dass** er im Vergleich zu dem Ausgangsstamm eine verminderte zelluläre Glutathion-Synthetase-Aktivität besitzt, und eine zelluläre γ-Glutamylcystein-Synthetase-Aktivität aufweist, die stärker erhöht ist als in einem Stamm mit gleichartig verminderter zellulärer Glutathion-Synthetase-Aktivität.

2. Mikroorganismenstamm gemäß Anspruch 1, **dadurch gekennzeichnet, dass** die Glutathion-Synthetase-Aktivität im erfindungsgemäßen Stamm soweit vermindert ist, dass sie maximal 50% der Glutathion-Synthetase-Aktivität eines entsprechenden Wildtypstammes beträgt.

3. Mikroorganismenstamm gemäß Anspruch 1 oder 2, **dadurch gekennzeichnet, dass** die zelluläre γ-Glutamylcystein-Synthetase-Aktivität um mindestens einen Faktor 5 höher ist, als in einem Stamm mit gleichartig verringerter zellulärer Glutathion-Synthetase-Aktivität.

4. Mikroorganismenstamm gemäß Anspruch 1, 2 oder 3, **dadurch gekennzeichnet, dass** der Ausgangsstamm der Spezies E-*scherichia coli* oder *Pantoea ananatis* angehört.

5. Mikroorganismenstamm gemäß einem der Ansprüche 1 bis 4, **dadurch gekennzeichnet, dass** der Ausgangsstamm ein Mikroorganismenstamm ist, der den Biosyntheseweg für γ-Glutamylcystein aufweist und eine, im Vergleich zu einem entsprechenden Ausgangsstamm erhöhte L-Cystein-Biosynthese-Leistung besitzt.

6. Mikroorganismenstamm gemäß einem der Ansprüche 1 bis 5,
**dadurch gekennzeichnet, dass** die Aktivitätserhöhung der γ-Glutamylcystein-Synthetase durch Erhöhung der Kopienzahl des gshA-Gens oder eines *gshA*-Homologs oder durch Einsatz eines Promotors, welcher zu einer verstärkten Expression von *gshA* führt, erreicht wird.

7. Mikroorganismenstamm gemäß einem der Ansprüche 1 bis 6, **dadurch gekennzeichnet, dass** die Kopienzahl von *gshA* oder eines *gshA*-Homologs durch Klonierung in Plasmid-Vektoren unter der Kontrolle eines Promotors erhöht ist.

8. Mikroorganismenstamm gemäß einem der Ansprüche 1 bis 7, **dadurch gekennzeichnet, dass** GshA eine Proteinsequenz aufweist, in der die Aminosäure Alanin an Position 494 gegen Valin oder Leucin (A494V oder A494L) ausgetauscht ist, oder die Aminosäure Serin an Position 495 gegen Threonin (S495T) ausgetauscht ist.

9. Mikroorganismenstamm gemäß einem der Ansprüche 1 bis 8, **dadurch gekennzeichnet, dass** das native Startcodon TTG des *gshA*-Gens gegen ATG ersetzt ist.

10. Verfahren zur Überproduktion von γ-Glutamylcystein (γGC) und den Derivaten dieses Dipeptids, γ-Glutamylcystin und bis-γ-Glutamylcystin, welches **dadurch gekennzeichnet ist, dass** ein Mikroorganismenstamm gemäß einem der Ansprüche 1 bis 9 in einem Fermentationsmedium kultiviert wird, die Zellen aus dem Medium entfernt werden und die gewünschten Produkte aus dem Kulturmedium aufgereinigt werden.


SEQUENCE LISTING

<110>  Consortium fuer elektrochemische Industrie - Wacker Chemie
       AG
       Thön, Dr. Marcel
       Schlösser, Dr. Thomas

<120>  Mikroorganismus und Verfahren zur fermentativen Überproduktion
       von Gamma-Glutamylcystein und Derivaten dieses Dipeptids

<130>  Co11216

<160>  24

<170>  PatentIn version 3.5

<210>  1
<211>  1557
<212>  DNA
<213>  Escherichia coli


<220>
<221>  gene
<222>  (1)..(1557)
<223>  gshA

<400>  1
ttgatcccgg acgtatcaca ggcgctggcc tggctggaaa aacatcctca ggcgttaaag      60

gggatacagc gtgggctgga gcgcgaaact ttgcgtgtta atgctgatgg cacactggca     120

acaacaggtc atcctgaagc attaggttcc gcactgacgc acaaatggat tactaccgat     180

tttgcggaag cattgctgga attcattaca ccagtggatg gtgatattga acatatgctg     240

acctttatgc gcgatctgca tcgttatacg gcgcgcaata tgggcgatga gcggatgtgg     300

ccgttaagta tgccatgcta catcgcagaa ggtcaggaca tcgaactggc acagtacggc     360

acttctaaca ccggacgctt taaaacgctg tatcgtgaag ggctgaaaaa tcgctacggc     420

gcgctgatgc aaaccatttc cggcgtgcac tacaatttct ctttgccaat ggcattctgg     480

caagcgaagt gcggtgatat ctcgggcgct gatgccaaag agaaaatttc tgcgggctat     540

ttccgcgtta tccgcaatta ctatcgtttc ggttgggtca ttccttatct gtttggtgca     600

tctccggcga tttgttcttc tttcctgcaa ggaaaaccaa cgtcgctgcc gtttgagaaa     660

accgagtgcg gtatgtatta cctgccgtat gcgacctctc ttcgtttgag cgatctcggc     720

tataccaata aatcgcaaag caatcttggt attaccttca acgatcttta cgagtacgta     780

gcgggcctta aacaggcaat caaaacgcca tcggaagagt acgcgaagat tggtattgag     840

aaagacggta agaggctgca aatcaacagc aacgtgttgc agattgaaaa cgaactgtac     900

gcgccgattc gtccaaaacg cgttacccgc agcggcgagt cgccttctga tgcgctgtta     960

cgtggcggca ttgaatatat tgaagtgcgt tcgctggaca tcaacccgtt ctcgccgatt    1020

ggtgtagatg aacagcaggt gcgattcctc gacctgttta tggtctggtg tgcgctggct    1080

gatgcaccgg aaatgagcag tagcgaactt gcctgtacac gcgttaactg gaaccgggtg      1140

atcctcgaag gtcgcaaacc gggtctgacg ctgggtatcg gctgcgaaac cgcacagttc      1200

ccgttaccgc aggtgggtaa agatctgttc cgcgatctga aacgcgtcgc gcaaacgctg      1260

gatagtatta acggcggcga agcgtatcag aaagtgtgtg atgaactggt tgcctgcttc      1320

gataatcccg atctgacttt ctctgcccgt atcttaaggt ctatgattga tactggtatt      1380

ggcggaacag gcaaagcatt tgcagaagcc taccgtaatc tgctgcgtga agagccgctg      1440

gaaattctgc gcgaagagga ttttgtagcc gagcgcgagg cgtctgaacg ccgtcagcag      1500

gaaatggaag ccgctgatac cgaaccgttt gcggtgtggc tggaaaaaca cgcctga         1557

<210>  2
<211>  518
<212>  PRT
<213>  Escherichia coli

<220>
<221>  PEPTIDE
<222>  (1)..(518)
<223>  GshA

<400>  2

Met Ile Pro Asp Val Ser Gln Ala Leu Ala Trp Leu Glu Lys His Pro
1               5                   10                  15

Gln Ala Leu Lys Gly Ile Gln Arg Gly Leu Glu Arg Glu Thr Leu Arg
            20                  25                  30

Val Asn Ala Asp Gly Thr Leu Ala Thr Thr Gly His Pro Glu Ala Leu
        35                  40                  45

Gly Ser Ala Leu Thr His Lys Trp Ile Thr Thr Asp Phe Ala Glu Ala
    50                  55                  60

Leu Leu Glu Phe Ile Thr Pro Val Asp Gly Asp Ile Glu His Met Leu
65                  70                  75                  80

Thr Phe Met Arg Asp Leu His Arg Tyr Thr Ala Arg Asn Met Gly Asp
                85                  90                  95

Glu Arg Met Trp Pro Leu Ser Met Pro Cys Tyr Ile Ala Glu Gly Gln
            100                 105                 110

Asp Ile Glu Leu Ala Gln Tyr Gly Thr Ser Asn Thr Gly Arg Phe Lys
        115                 120                 125

Thr Leu Tyr Arg Glu Gly Leu Lys Asn Arg Tyr Gly Ala Leu Met Gln
    130                 135                 140

Thr Ile Ser Gly Val His Tyr Asn Phe Ser Leu Pro Met Ala Phe Trp
145                 150                 155                 160

Gln Ala Lys Cys Gly Asp Ile Ser Gly Ala Asp Ala Lys Glu Lys Ile
                165                 170                 175

Ser Ala Gly Tyr Phe Arg Val Ile Arg Asn Tyr Tyr Arg Phe Gly Trp
            180                 185                 190

Val Ile Pro Tyr Leu Phe Gly Ala Ser Pro Ala Ile Cys Ser Ser Phe
        195                 200                 205

Leu Gln Gly Lys Pro Thr Ser Leu Pro Phe Glu Lys Thr Glu Cys Gly
    210                 215                 220

Met Tyr Tyr Leu Pro Tyr Ala Thr Ser Leu Arg Leu Ser Asp Leu Gly
225                 230                 235                 240

Tyr Thr Asn Lys Ser Gln Ser Asn Leu Gly Ile Thr Phe Asn Asp Leu
                245                 250                 255

Tyr Glu Tyr Val Ala Gly Leu Lys Gln Ala Ile Lys Thr Pro Ser Glu
            260                 265                 270

Glu Tyr Ala Lys Ile Gly Ile Glu Lys Asp Gly Lys Arg Leu Gln Ile
        275                 280                 285

Asn Ser Asn Val Leu Gln Ile Glu Asn Glu Leu Tyr Ala Pro Ile Arg
    290                 295                 300

Pro Lys Arg Val Thr Arg Ser Gly Glu Ser Pro Ser Asp Ala Leu Leu
305                 310                 315                 320

Arg Gly Gly Ile Glu Tyr Ile Glu Val Arg Ser Leu Asp Ile Asn Pro
                325                 330                 335

Phe Ser Pro Ile Gly Val Asp Glu Gln Gln Val Arg Phe Leu Asp Leu
            340                 345                 350

Phe Met Val Trp Cys Ala Leu Ala Asp Ala Pro Glu Met Ser Ser Ser
        355                 360                 365

Glu Leu Ala Cys Thr Arg Val Asn Trp Asn Arg Val Ile Leu Glu Gly
    370                 375                 380

Arg Lys Pro Gly Leu Thr Leu Gly Ile Gly Cys Glu Thr Ala Gln Phe
385                 390                 395                 400

Pro Leu Pro Gln Val Gly Lys Asp Leu Phe Arg Asp Leu Lys Arg Val
                405                 410                 415

Ala Gln Thr Leu Asp Ser Ile Asn Gly Gly Glu Ala Tyr Gln Lys Val
            420                 425                 430

Cys Asp Glu Leu Val Ala Cys Phe Asp Asn Pro Asp Leu Thr Phe Ser
        435                 440                 445

Ala Arg Ile Leu Arg Ser Met Ile Asp Thr Gly Ile Gly Gly Thr Gly
    450                 455                 460

Lys Ala Phe Ala Glu Ala Tyr Arg Asn Leu Leu Arg Glu Glu Pro Leu
465                 470                 475                 480

Glu Ile Leu Arg Glu Glu Asp Phe Val Ala Glu Arg Glu Ala Ser Glu
                485                 490                 495

Arg Arg Gln Gln Glu Met Glu Ala Ala Asp Thr Glu Pro Phe Ala Val
            500                 505                 510

Trp Leu Glu Lys His Ala
        515

<210>  3
<211>  951
<212>  DNA
<213>  Escherichia coli

<220>
<221>  gene
<222>  (1)..(951)
<223>  gshB

<400>  3
atgatcaagc tcggcatcgt gatggacccc atcgcaaaca tcaacatcaa gaaagattcc      60

agttttgcta tgttgctgga agcacagcgt cgtggttacg aacttcacta tatggagatg     120

ggcgatctgt atctgatcaa tggtgaagcc cgcgcccata cccgcacgct gaacgtgaag     180

cagaactacg aagagtggtt ttcgttcgtc ggtgaacagg atctgccgct ggccgatctc     240

gatgtgatcc tgatgcgtaa agacccgccg tttgataccg agtttatcta cgcgacctat     300

attctggaac gtgccgaaga gaaagggacg ctgatcgtta acaagccgca gagcctgcgc     360

gactgtaacg agaaactgtt taccgcctgg ttctctgact taacgccaga aacgctggtt     420

acgcgcaata aagcgcagct aaaagcgttc tgggagaaac acagcgacat cattcttaag 480

ccgctggacg gtatgggcgg cgcgtcgatt ttccgcgtga aagaaggcga tccaaacctc 540

ggcgtgattg ccgaaaccct gactgagcat ggcactcgct actgcatggc gcaaaattac 600

ctgccagcca ttaaagatgg cgacaaacgc gtgctggtgg tggatggcga gccggtaccg 660

tactgcctgg cgcgtattcc gcaggggggc gaaacccgtg gcaatctggc tgccggtggt 720

cgcggtgaac ctcgtccgct gacggaaagt gactggaaaa tcgcccgtca gatcgggccg 780

acgctgaaag aaaaagggct gatttttgtt ggtctggata tcatcggcga ccgtctgact 840

gaaattaacg tcaccagccc aacctgtatt cgtgagattg aagcagagtt tccggtgtcg 900

atcaccggaa tgttaatgga tgccatcgaa gcacgtttac agcagcagta a 951

<210> 4
<211> 316
<212> PRT
<213> Escherichia coli

<220>
<221> PEPTIDE
<222> (1)..(316)
<223> GshB

<400> 4

<210>  5
<211>  70
<212>  DNA
<213>  Artificial Sequence

<220>
<223>  Oligonukleotid: gshB-del-for

<400>  5
atgatcaagc tcggcatcgt gatggacccc atcgcaaaca tcaacatcaa gtgtaggctg        60

gagctgcttc                                                               70

<210>  6
<211>  70
<212>  DNA
<213>  Artificial Sequence

<220>
<223>  Oligonukleotid: gshB-del-rev

<400>  6
ttactgctgc tgtaaacgtg cttcgatggc atccattaac attccggtga ccgtcgacct      60

gcagttcgaa                                                            70


<210>  7
<211>  30
<212>  DNA
<213>  Artificial Sequence

<220>
<223>  Oligonukleotid: gshAp-gshA-for

<400>  7
agtctctaga acgttatcct tgggcgtttc                                      30


<210>  8
<211>  32
<212>  DNA
<213>  Artificial Sequence

<220>
<223>  Oligonukleotid: gshAp-gshA-rev

<400>  8
agcttctaga ttgtaggcct gcacatatgg tc                                   32


<210>  9
<211>  29
<212>  DNA
<213>  Artificial Sequence

<220>
<223>  Oligonukleotid: gshA-OE-for

<400>  9
cccgaattca tgatcccgga cgtatcaca                                       29


<210>  10
<211>  29
<212>  DNA
<213>  Artificial Sequence

<220>
<223>  Oligonukleotid: gshA-OE-rev

<400>  10
aaaagatctt caggcgtgtt tttccagcc                                       29


<210>  11

<211>  32
<212>  DNA
<213>  Artificial Sequence

<220>
<223>  Oligonukleotid: gshA-EcoRImut-for

<400>  11
tcaccatcca ctggtgtaat aaattccagc aa                                    32


<210>  12
<211>  30
<212>  DNA
<213>  Artificial Sequence

<220>
<223>  Oligonukleotid: gshA-EcoRImut-rev

<400>  12
attacaccag tggatggtga tattgaacat                                       30


<210>  13
<211>  32
<212>  DNA
<213>  Artificial Sequence

<220>
<223>  Oligonukleotid: gshA-BglIImut-for

<400>  13
cgtttcagat cgcggaacag gtctttaccc ac                                    32


<210>  14
<211>  30
<212>  DNA
<213>  Artificial Sequence

<220>
<223>  Oligonukleotid: gshA-BglIImut-rev

<400>  14
ctgttccgcg atctgaaacg cgtcgcgcaa                                       30


<210>  15
<211>  31
<212>  DNA
<213>  Artificial Sequence

<220>
<223>  Oligonukleotid: tufBp-for

<400>  15
agtctctaga caatgatgtt gaaaaagtgt g                                     31


<210>  16
<211>  49
<212>  DNA
<213>  Artificial Sequence

<220>
<223>  Oliginukleotid: tufBp-rev

<400>  16
agcttctaga agatcttacg gcgaattcca agtagcgcgc attctatgg                    49


<210>  17
<211>  36
<212>  DNA
<213>  Artificial Sequence

<220>
<223>  Oligonukleotid: tufBp-RBSopt-for

<400>  17
gaagatctta cggcgaattc ctcctgcgcg cattct                                  36


<210>  18
<211>  30
<212>  DNA
<213>  Artificial Sequence

<220>
<223>  Oligonukleotid: tufBp-RBSopt-rev

<400>  18
gaattcgccg taagatcttc tagatttcag                                         30


<210>  19
<211>  32
<212>  DNA
<213>  Artificial Sequence

<220>
<223>  Oligonukleotid: tufBp-10opt-for

<400>  19
gaattcctcc tgcgcgcatt atatggagac at                                      32


<210>  20
<211>  30
<212>  DNA
<213>  Artificial Sequence

<220>
<223>  Oligonukleotid: tufBp-10opt-rev

<400>  20
aatgcgcgca ggaggaattc gccgtaagat                                         30


<210>  21
<211>  34
<212>  DNA
<213>  Artificial Sequence

<220>
<223>  Oligonukleotid: tufBp-35opt-for

<400>  21
atatggagac atgcgagttc tgtcaactaa aaaa                                    34


<210>  22
<211>  30
<212>  DNA
<213>  Artificial Sequence

<220>
<223>  Oligonukleotid: tufBp-35opt-rev

<400>  22
gaactcgcat gtctccatat aatgcgcgca                                         30


<210>  23
<211>  27
<212>  DNA
<213>  Artificial Sequence

<220>
<223>  Oligonukleotid: serA-NcoI-for

<400>  23
agtcccatgg cgcagatgaa atcaacg                                            27


<210>  24
<211>  30
<212>  DNA
<213>  Artificial Sequence

<220>
<223>  Oligonukleotid: serA-SacI-rev

<400>  24
tcaggagctc ttagtacagc agacgggcgc                                         30